# EUROPÄISCHE PATENTSCHRIFT

(11) **EP 2 025 319 B1**
(45) Veröffentlichungstag und Bekanntmachung des Hinweises auf die Patenterteilung: **23.09.2015**
(21) Anmeldenummer: 08005431.5
(22) Anmeldetag: 22.03.2008
(51) Int. Cl.: A61K 8/19, A61K 8/55, A61Q 11/00

(54) **Desensibilisierende Mund- und Zahnpflege- und reinigungsmittel**
Numbing oral and tooth care and cleaning agents
Produit de nettoyage et de soin désensibilisant pour la bouche et les dents

(30) Priorität: 13.08.2007 DE 102007038259
(43) Veröffentlichungstag der Anmeldung: 18.02.2009
(73) Patentinhaber: Henkel AG & Co. KGaA, 40589 Düsseldorf (DE)
(72) Erfinder: Barth, Adolf Peter, 42781 Haan (DE); Leinen, Hans-Theo, 40699 Erkrath (DE); Bernecker, Ullrich, 52074 Aachen (DE); Adelmann, Barbara, 40597 Düsseldorf (DE); Stade, Daniela, 40595 Düseldorf (DE)

(56) Entgegenhaltungen:
- EP-A1- 0 988 857
- WO-A1-96/20693
- WO-A1-2007/051543
- WO-A1-2008/095202
- WO-A2-2007/085428
- GB-A- 2 188 548
- JP-A- 2006 325 455
- US-A- 4 397 837
- US-B1- 6 475 539
- US-B1- 6 491 899
- ANONYMOUS: "Unique Sensitive Teeth Formula, Complete Protection for your Teeth & Gums" DENTONIC PRODUCT, [Online] 3. September 2006 (2006-09-03), XP002512418 Gefunden im Internet: URL:http://web.archive.org/web/20060903003 422/http://www.dentonic.com.pk/products/de ntonicsensitive.asp> [gefunden am 2009-01-27]

## Beschreibung

Die Erfindung betrifft Mund- und Zahnpflegemittel mit einer Wirkstoffkombination zur Verringerung der Sensibilität der Zähne gegen mechanische und thermische Reize, die gleichzeitig die Zahnoberflächen gründlich reinigt und Mundgeruch verringern hilft.

Zahnreinigungsmittel sind in verschiedenen Formen auf dem Markt und dienen in erster Linie der Reinigung der Zahnoberfläche und der Vorbeugung von Zahn- und Zahnfleischerkrankungen. Sie enthalten üblicherweise eine Kombination aus Poliermitteln, Feuchthaltemitteln, Tensiden, Bindemitteln, Aromastoffen und fluoridhaltigen sowie antimikrobiellen Wirkstoffen. Neben Zahnpulvern, die wegen ihrer erhöhten Abrasivität eine untergeordnete Rolle spielen, werden Zahnreinigungsmittel vor allem in Pasten-, Creme- und transluzenter oder transparenter Gelform angeboten. In den letzten Jahren haben auch Liquid- oder Flüssigzahncremes und Mundwässer zunehmend an Bedeutung gewonnen.

Neben der Reinigung der Zähne erwartet der Verbraucher von den gattungsgemäßen Produkten auch eine Pflege der Zähne und der Mundhöhle. So sind insbesondere ein "sauberes" Gefühl, d.h. eine glatte und glänzende Zahnoberfläche sowie ein frisches Gefühl im Mund wesentliche Aspekte für den Kaufanreiz von Zubereitungen zur Mund- und Zahnpflege- und -reinigung. Ein erfolgreiches Mittel der gattungsgemäßen Art sollte daher die Zähne gründlich reinigen, ohne den Zahn oder die Zahnoberfläche zu schädigen und gleichzeitig Mundgeruch verringern und/oder verhindern.

Mundgeruch, auch Halitosis oder "Foetor ex ore" genannt, kann verschiedene Ursachen haben. Bei gesunden Menschen wird Mundgeruch jedoch in den meisten Fällen durch Bakterien im Mund- bzw. Rachenraum hervorgerufen, die Körperzellen oder Nahrungsreste verstoffwechseln, wobei als Abbauprodukte von Proteinen u.a. flüchtige Schwefelverbindungen ("volatile sulfur compounds", VSC) entstehen, die für den üblen Geruch verantwortlich gemacht werden.

Diese Schwefelverbindungen sind insbesondere: H₂S = Schwefelwasserstoff (zu etwa 30 %), CH₃-S-H = Methylmercaptan (zu etwa 60 %) und CH₃-S-CH₃ = Dimethylsulfid (zu etwa 10 %).

Die flüchtigen Schwefelverbindungen sind teilweise sehr aggressiv und können das Zahnfleisch und die Mundschleimhaut schädigen. So ist beispielsweise bekannt, daß Schwefelwasserstoff und Methylmercaptan die Durchlässigkeit der Mundschleimhaut erhöhen und so Zahnfleischerkrankungen Vorschub leisten können.

Zahnfleischerkrankungen stellen einen hohen Risikofaktor für die Gesundheit dar. Nach Untersuchungen der Weltgesundheitsorganisation (WHO) leiden über 50 % der erwachsenen Bundesbürger an dringend behandlungsbedürftigen Parodontopathien.

Zahnfleischerkrankungen werden im Volksmund oft "Parodontose" genannt. Dieser Ausdruck ist nicht korrekt, da die Endung "-ose" einen normalen, altersbedingten Rückgang eines Organs beschreibt. Richtig sind die Ausdrücke "Parodontitis" oder auch "entzündliche Parodontopathie". Sie ist neben der Karies die weit verbreiteste Erkrankung der Mundhöhle und tritt hauptsächlich bei Erwachsenen auf.

Unter Parodontitis versteht man eine durch Bakterien hervorgerufene entzündliche Veränderung des den Zahn umgebenden Gewebes, besonders des Kieferknochens. Eine Parodontitis (= mit Beteiligung der Zahnfleischtaschen und des Knochens) entwickelt sich immer aus einer Zahnfleischentzündung (Gingivitis) und befällt zuerst die der Reinigung am schlechtesten zugänglichen Zahnzwischenräume. Die sich in der Zahnfleischtasche befindlichen Bakterien wirken durch ihre Stoffwechselprodukte, insbesondere durch die von Ihnen produzierten VSC, destruktiv auf Zahnfleisch, Zahnsubstanz und Knochen ein und verstärken so ständig die bereits bestehende Parodontitis.

Dieses gesundheitliche Problem ist sehr weit verbreitet: Untersuchungen zeigen, dass etwa ab dem 35. Lebensjahr mehr Zähne durch Parodontopathien (Zahnfleischerkrankungen) als durch Karies verloren gehen.

Es hat nicht an Versuchen gefehlt, Mundgeruch zu bekämpfen, und eine Vielzahl von Agentien wird zu diesem Zweck in Zubereitungen zur Mund- und Zahnpflege und -reinigung eingesetzt. Als antibakterielle Mittel finden beispielsweise Triclosan, Zinnsalze oder Chlorhexidin Verwendung.

Der Einsatz dieser Produkte kann jedoch in Einzelfällen mit Nachteilen verbunden sein, die teils geschmacklicher Natur sind, teils ästhetische Gründe haben, da das Produkt und/oder die Zähne durch übermäßigen Gebrauch der Agentien verfärbt werden können.

Es besteht daher nach wie vor das Bedürfnis, Halitosis zu bekämpfen und Wirkstoffe oder Wirkstoffkombinationen dafür bereitzustellen, die frei von den genannten Nachteilen sind.

Manche Menschen leiden unter einer extremen Empfindlichkeit der Zähne, insbesondere im Bereich der Zahnhälse und verspüren ein unangenehmes Gefühl bis hin zum Schmerz, wenn die Zähne z.B. beim Genuß gekühlter Speisen oder Getränke oder bei der Berührung mit der Zahnbürste einem thermischen oder mechanischen Reiz ausgesetzt werden.

Es ist bekannt, daß wasserlösliche Salze des Kaliums und Strontiums diese Unempfindlichkeit in gewissem Umfang verringern können. So wird z.B. in DE 24 19 384 ein Zusatz von Kaliumnitrat, in US 3,122,483 der Zusatz von Strontiumchlorid zu Zahnpasten empfohlen.

Die Aufgabe der vorliegenden Erfindung bestand darin, Zubereitungen zur Mund- und Zahnpflege und -reinigung bereitzustellen, die die Zähne desensibilisieren und zudem Mundgeruch wirksam bekämpfen und gegen Gingivitis und Parodontitis wirksam sind.

Darüber hinaus lag die Aufgabe darin, auch weitergehenden Nutzen in die erfindungsgemäßen Mittel einzubringen. So sollte insbesondere die Prophylaxe vor Karies, die Behandlung von Oberflächendefekten auf der Zahnoberfläche und damit die Behandlung von Überempfindlichkeit der Zähne durch den Einsatz der erfindungsgemäßen Mittel ermöglicht werden.

Es wurde nun aber gefunden, daß ein Gehalt an Calcium-Glycerophosphat die desensibilisierende Wirkung von Kaliumsalzen auf überempfindliche Zähne noch erheblich steigert. Insbesondere wurde ein rascheres Eintreten der Wirkung beobachtet. Eine solche, im Zusammenwirken mit anderen desensibilisierend wirkenden Stoffen potenzierende Wirkung des Claciumglycerophosphats war bisher unbekannt.

Gegenstand der Erfindung sind daher Mund- und Zahnpflege- und -reinigungsmittel, enthaltend - bezogen auf sein Gewicht -
a) 0,1 bis 1 Gew.-% Calcium-Glycerophosphat;
b) 3 bis 7 Gew.-% mindestens eines Kaliumsalzes,

Mund- und Zahnpflegemittel sowie Mund- und Zahnreinigungsmittel im Sinne der Erfindung sind Mund- und Zahnpulver, Mund- und Zahnpasten, flüssige Mund- und Zahncremes sowie Mund- und Zahngele. Bevorzugt geeignet sind Zahnpasten und flüssige Zahnreinigungsmittel. Hierzu können die Mund- und Zahnpflege- und reinigungsmittel z.B. in Form von Zahnpasten, flüssigen Zahncremes, Zahnpulvern, Mundwässern oder gegebenenfalls auch als Kaumasse, z. B. als Kaugummi, vorliegen. Bevorzugt liegen sie jedoch als mehr oder weniger fließfähige oder plastische Zahnpasten vor, wie sie zur Reinigung der Zähne unter Einsatz einer Zahnbürste verwendet werden.

Als ersten wesentlichen Bestandteil enthalten die erfindungsgemäßen Mund und Zahnpflege- und
- reinigungsmittel 0,1 bis 1 Gew.-% Calcium-Glycerophosphat, d.h. mindestens eines Calciumsalzes mindestens einer Glycerophosphorsäure.
Die Glycerophosphorsäure ist eine zweibasige Säure, die in zwei isomeren Formen vorkommt, je nachdem, ob die Phosphorsäuregruppierung an eine terminale oder die mediale OH-Gruppe des Glycerins gebunden vorliegt. Die Form, bei der die Phosphorsäuregruppierung an eine terminale OH-Gruppe des Glycerins gebunden vorliegt, wird auch als alpha-Isomer, die Form, bei der die Phosphorsäuregruppierung an die mediale OH-Gruppe des Glycerins gebunden vorliegt, auch als beta-Isomer bezeichnet.
Das alpha-Isomer ist zusätzlich optisch aktiv und tritt in den zwei enantiomeren Formen sn-Glycerol-1-phosphorsäure 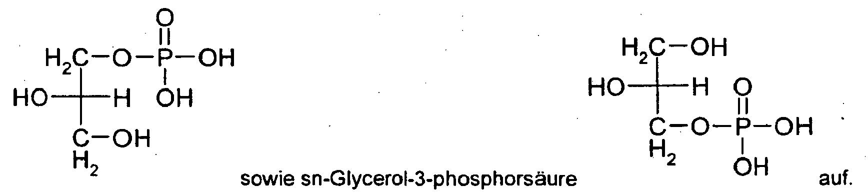 Das Präfix sn bei Glycerol-Derivaten steht für "stereospezifisch nummeriert" und verlangt, daß die 2-Hydroxy-Gruppe in der vorstehend verwendeten Fischer-Projektion nach links weist. Glycerol-2-phosphat ist nicht optisch aktiv. Die Glycerophosphorsäuren sind etwa so stark wie Phosphorsäure.
Erfindungsgemäß bevorzugt ist der Einsatz des alpha-Isomers, unabhängig davon, welches der beiden Enantiomere eingesetzt wird. Sofern der Einsatz enantiomerenreiner Verbindungen gewünscht ist, wird vorzugsweise das Calciumsalz der sn-Glycerol-3-phosphorsäure eingesetzt.
Zusammenfassend sind erfindungsgemäße Mund und Zahnpflege- und -reinigungsmittel bevorzugt, die Calciumsalze der Glycerophosphorsäuren der Formeln (la) und (Ib) enthalten

HO-CH₂-CH(OH)-CH₂OP(O)O₂²⁺ (Ia).

HO-CH₂-CH(OP(O)O₂²⁻)-CH₂-OH Ca²⁺ (Ib).

Hierbei sind besonders bevorzugte erfindungsgemäße Mund und Zahnpflege- und reinigungsmittel dadurch gekennzeichnet, daß das Gewichtsverhältnis der Calciumsalze der Formeln (Ia) zu (Ib) oberhalb von 50:50, vorzugsweise oberhalb von 60:40, besonders bevorzugt oberhalb von 70:30 und insbesondere oberhalb 80:20 liegt.
Der Einsatz der Calcium-Glycerophosphate innerhalb engerer Mengenbereiche ist bevorzugt. Bevorzugte erfindungsgemäße Mund- und Zahnpflege- und -reinigungsmittel enthalten - bezogen auf ihr Gewicht bevorzugt 0,1 bis 1,0 Gew.-%, weiter bevorzugt 0,11 bis 0,75 Gew.-% und insbesondere 0,12 bis 0,5 Gew.-% Calcium-Glycerophosphat.
Als zweiten wesentlichen Bestandteil enthalten die erfindungsgemäßen Mittel 3 bis 7 Gew.-% mindestens eines Kaliumsalzes. Der Einsatz der Kaliumsalze innerhalb engerer Mengenbereiche ist bevorzugt. Bevorzugte erfindungsgemäße Mund und Zahnpflege- und- reinigungsmittel sind dadurch gekennzeichnet, daß sie 4 bis 6 Gew.-% Kaliumsalz(e) enthalten.
Obwohl hinsichtlich der Auswahl der Kaliumsalze keine Einschränkungen bestehen, sind bevorzugte Kaliumsalze gut wasserlöslich. Besonders bevorzugte erfindungsgemäße Mund und Zahnpflege- und -reinigungsmittel sind dabei dadurch gekennzeichnet, daß sie ausschließlich Kaliumsalz(e) enthalten, welche(s) bei 20°C eine Löslichkeit in destilliertem Wasser oberhalb von 200 g/l, vorzugsweise oberhalb von 250 g/l und insbesondere oberhalb von 300 g/l aufweisen.
Geeignete wasserlösliche Salze des Kaliums sind z.B. Kaliumchlorid, Kaliumsulfat, Kaliumbicarbonat, Kaliumcitrat, Kaliumacetat, Kaliumlactat und Kaliumnitrat. Bevorzugt eignet sich Kaliumnitrat.
Es hat sich gezeigt, daß die desensibilisierende Wirkung von Kaliumsalzen auf überempfindliche Zähne durch den Einsatz von Calciumglycerophosphat gesteigert wird. Die Steigerung ist besonders innerhalb bestimmter Gewichtsverhältnisse der Komponenten zueinander zu beobachten. Bei bevorzugten erfindungsgemäßen Mund und Zahnpflege- und -reinigungsmitteln beträgt das Gewichtsverhältnis zwischen Calcium-Glycerophosphat und der Gesamtmenge an Kaliumsalz(en) 1 : 1000 bis 1 : 1, vorzugsweise 0,5 : 100 bis 75 : 100, weiter bevorzugt 1 : 100 bis 50 : 100,noch weiter bevorzugt 1,5 : 100 bis 10 : 100 und insbesondere 2 : 100 bis 3 : 100.
Besonders bevorzugte erfindungsgemäße Mittel enthalten 0,1 bis 0,25 Gew.-% Calcium-Glycerophosphat und 4 bis 6 Gew.-% Kaliumnitrat, wobei Mittel, die folgende Kombinationen enthalten, besonders bevorzugt sind:

| **Calcium-Glycerophosphat** **[Gew.-%, bezogen auf gesamtes Mittel]** | **Kaliumnitrat** **[Gew.-%, bezogen auf gesamtes Mittel]** |
|---|---|
| 0,10 | 4,0 |
| 0,10 | 4,1 |
| 0,10 | 4,2 |
| 0,10 | 4,3 |
| 0,10 | 4,4 |
| 0,10 | 4,5 |
| 0,10 | 4,6 |
| 0,10 | 4,7 |
| 0,10 | 4,8 |
| 0,10 | 5,0 |
| 0,10 | 5,1 |
| 0,10 | 5,2 |
| 0,10 | 5,3 |
| 0,10 | 5,4 |
| 0,10 | 5,5 |
| 0,10 | 5,6 |
| 0,10 | 5,7 |
| 0,10 | 5,8 |
| 0,10 | 5,9 |
| 0,10 | 6,0 |
| 0,11 | 4,0 |
| 0,11 | 4,1 |
| 0,11 | 4,2 |
| 0,11 | 4,3 |
| 0,11 | 4,4 |
| 0,11 | 4,5 |
| 0,11 | 4,6 |
| 0,11 | 4,7 |
| 0,11 | 4,8 |
| 0,11 | 5,0 |
| 0,11 | 5,1 |
| 0,11 | 5,2 |
| 0,11 | 5,3 |
| 0,11 | 5,4 |
| 0,11 | 5,5 |
| 0,11 | 5,6 |
| 0,11 | 5,7 |
| 0,11 | 5,8 |
| 0,11 | 5,9 |
| 0,11 | 6,0 |
| 0,12 | 4,0 |
| 0,12 | 4,1 |
| 0,12 | 4,2 |
| 0,12 | 4,3 |
| 0,12 | 4,4 |
| 0,12 | 4,5 |
| 0,12 | 4,6 |
| 0,12 | 4,7 |
| 0,12 | 4,8 |
| 0,12 | 5,0 |
| 0,12 | 5,1 |
| 0,12 | 5,2 |
| 0,12 | 5,3 |
| 0,12 | 5,4 |
| 0,12 | 5,5 |
| 0,12 | 5,6 |
| 0,12 | 5,7 |
| 0,12 | 5,8 |
| 0,12 | 5,9 |
| 0,12 | 6,0 |
| 0,13 | 4,0 |
| 0,13 | 4,1 |
| 0,13 | 4,2 |
| 0,13 | 4,3 |
| 0,13 | 4,4 |
| 0,13 | 4,5 |
| 0,13 | 4,6 |
| 0,13 | 4,7 |
| 0,13 | 4,8 |
| 0,13 | 5,0 |
| 0,13 | 5,1 |
| 0,13 | 5,2 |
| 0,13 | 5,3 |
| 0,13 | 5,4 |
| 0,13 | 5,5 |
| 0,13 | 5,6 |
| 0,13 | 5,7 |
| 0,13 | 5.8 |
| 0,13 | 5,9 |
| 0,13 | 6,0 |
| 0,14 | 4,0 |
| 0,14 | 4,1 |
| 0,14 | 4,2 |
| 0,14 | 4,3 |
| 0,14 | 4,4 |
| 0,14 | 4,5 |
| 0,14 | 4,6 |
| 0,14 | 4,7 |
| 0,14 | 4,8 |
| 0,14 | 5,0 |
| 0,14 | 5,1 |
| 0,14 | 5,2 |
| 0,14 | 5,3 |
| 0,14 | 5,4 |
| 0,14 | 5,5 |
| 0,14 | 5,6 |
| 0,14 | 5,7 |
| 0,14 | 5,8 |
| 0,14 | 5,9 |
| 0,14 | 6,0 |
| 0,15 | 4,0 |
| 0,15 | 4,1 |
| 0,15 | 4,2 |
| 0,15 | 4,3 |
| 0,15 | 4,4 |
| 0,15 | 4,5 |
| 0,15 | 4,6 |
| 0,15 | 4,7 |
| 0,15 | 4,8 |
| 0,15 | 5,0 |
| 0,15 | 5,1 |
| 0,15 | 5,2 |
| 0,15 | 5,3 |
| 0,15 | 5,4 |
| 0,15 | 5,5 |
| 0,15 | 5,6 |
| 0,15 | 5,7 |
| 0,15 | 5,8 |
| 0,15 | 5,9 |
| 0,15 | 6,0 |

Zusätzlich zu den Kaliumsalzen können die erfindungsgemäßen Mittel auch Strontiumsalze enthalten. Geeignete wasserlösliche Salze des Strontiums sind Strontiumchlorid, Strontiumnitrat, Strontiumcitrat, Strontiumacetat und Strontiumlactat.
Die erfindungsgemäße Kombination gegen empfindliche Zähne ist dann besonders effektiv, wenn auch der Wassergehalt der erfindungsgemäßen Mittel speziell auf sie abgestimmt wird. Insbesondere in wasserärmeren Formulierungen ist der Effekt der Desensibilisierung besonders ausgeprägt. Hier sind bevorzugte erfindungsgemäße Mund und Zahnpflege- und -reinigungsmittel dadurch gekennzeichnet, daß sie weniger als 55 Gew.-%, besonders bevorzugt weniger als 50 Gew.-% und insbesondere weniger als 45 Gew.-% Wasser enthalten.

Als weiterer Inhaltstoff, der die Empfindlichkeit der Zähne in Kombination mit dem Kaliumsalz und dem Calcium-Glycdrophosphat noch weiter senken kann, hat sich Eugneol bzw. Nelkenknospenöl bewährt. Nelkenknospenöl enthält in der Regel 70 - 90 Gew.-% Eugenol neben 10 - 15 Gew.-% Eugenolacetat (Geruchskomponente) sowie kleinere Anteile an Alkoholen, Methylestern, Ketonen und Sesquiterpenen.

Bevorzugte erfindungsgemäße Mund und Zahnpflege- und -reinigungsmittel enthalten zusätzlich 0,01 bis 1 Gew.-%, vorzugsweise 0,025 bis 0,5 Gew.-% und insbesondere 0,05 bis 0,2 Gew.-% Eugenol und/oder Nelkenknospenöl.

Die erfindungsgemäßen Mittel sind an sich bereits wirksam gegen Mundgeruch (Halitosis) sowie Parodontitis und/oder Gingivitis. Zur Steigerung dieser Effekte können sie weitere entzündungshemmende Substanzen enthalten.

Die erfindungsgemäßen Mittel, insbesondere die Zahnpasten, können auch zusätzlich weitere wundheilende und entzündungshemmende Stoffe, z. B. Wirkstoffe gegen Zahnfleischentzündungen, enthalten. Derartige Stoffe können z. B. ausgewählt sein aus Allantoin, Azulen, Kamillenextrakten, Tocopherol, Panthenol, Bisabolol, Salbeiextrakten.

Als nicht-kationische, bakterizide Komponente eignen sich z.B. Phenole, Resorcine, Bisphenole, Salicylanilide und deren halogenierte Derivate, halogenierte Carbanilide und p-Hydroxybenzoesäureester. Besonders bevorzugte antimikrobielle Komponenten sind halogenierte Diphenylether, z.B. 2,4-Dichlor-2'-hydroxydiphenylether, 4,4'-Dichlor-2'-hydroxydiphenylether, 2,4,4'-Tribrom-2'-hydroxydiphenylether und 2,4,4'-Trichlor-2'-hydroxydiphenylether (Triclosan). Sie werden bevorzugt in Mengen von 0,01 - 1 Gew.-% in die erfindungsgemäßen Zahnpflegemittel eingesetzt. Besonders bevorzugt wird Triclosan in einer Menge von 0,01 - 0,3 Gew.-% eingesetzt.

Bevorzugte erfindungsgemäße Mund und Zahnpflege- und -reinigungsmittel enthalten zur Verbesserung der entzündungshemmenden Eigenschaften eine Kombination von
- Panthenol oder einem Salz der Pantothensäure und
- Retinol oder einem Derivat davon.

D-Panthenol D - (+) - 2,4-Dihydroxy-N-(3-hydroxypropyl)-3,3-dimethyl-butyramid zeigt eine der Pantothensäure entsprechende biologische Aktivität. Die Pantothensäure (R - (+) - N - (2,4-Dihydroxy-3,3-dimethylbutyryl-β-alanin) ist eine Vorstufe in der Biosynthese des Coenzyms A und wird zum Vitamin-B-Komplex (B3) gezählt. Diese Stoffe sind dafür bekannt, daß sie die Wundheilung fördern und eine günstige Wirkung auf die Haut haben. Sie sind daher auch gelegentlich in Zahnpasten beschrieben worden. Die erfindungsgemäßen Zahnpflegemittel enthalten bevorzugt 0,05 - 5 Gew.-% Panthenol oder ein Salz der Pantothensäure.

Retinol (3,7-Dimethyl-9-(2,6,6-trimethyl-1-cyclohexenyl)-2,4,6,8-nonatetraen-1-ol ist der internationale Freiname für Vitamin A1. Anstelle des Retinols kann auch eines seiner Derivate mit ähnlicher biologischer Wirkung, z.B. ein Ester oder die Retinoesäure (Tretinoin), eines ihrer Salze oder ihre Ester verwendet werden. Bevorzugt wird ein Retinol-Ester, insbesondere ein Fettsäureester einer Fettsäure mit 12 - 22 C-Atomen verwendet. Besonders bevorzugt ist Retinol-Palmitat geeignet. Bei Verwendung eines Retinol-Esters, z.B. Retinol-Palmitat mit einer Aktivität von 1,7 ˙ 10⁶ I.E. pro g ist eine Menge von 0,001 bis 0,1 Gew.-% bevorzugt. Bei Verwendung anderer Retinol-Derivate empfiehlt sich eine Einsatzmenge, die einer Konzentration von 10³ bis 10⁶ I.E. (Internationale Einheiten) pro 100 g entspricht.

Bevorzugte Zahnpflegemittel gemäß der vorliegenden Erfindung enthalten neben Poliermitteln, Fluorverbindungen, Feuchthaltemitteln und Bindemitteln bevorzugt
0,01 - 1 Gew.-% eines halogenierten Diphenylethers
0,05 - 5 Gew.-% Panthenol oder ein Salz der Pantothensäure und
0,01 - 0,1 Gew.-% eines Retinol-Esters, bevorzugt Retinol-Palmitat.

Die erfindungsgemäßen Mund- und Zahnpflege- und -reinigungsmittel enthalten weitere I nhaltsstoffe.

Bevorzugt ist hierbei der Einsatz von so genannten Feuchthaltemitteln, die bei Zahnpasten das Austrocknen verhindern. Bei so genannten Flüssigzahncremes mit fließfähiger Rheologie dienen diese als Matrix und werden in höheren Mengen eingesetzt.

Hier sind erfindungsgemäße Mund- und Zahnpflege- und -reinigungsmittel bevorzugt, die - bezogen auf ihr Gewicht - 10 bis 60 Gew.-%, vorzugsweise 12,5 bis 50 Gew.-%, besonders bevorzugt 15 bis 45 Gew.-% und insbesondere 20 bis 40 Gew.-% mindestens eines mehrwertigen Alkohols aus der Gruppe Sorbit und/oder Glycerin und/oder 1,2-Propylenglycol.-% enthalten.

Sorbit (auch als Glucit bezeichnet) ist ein Zuckeralkohol von Glucose, also ein Hexit. Sorbit ist durch Hydrierung von Glucose herstellbar, spaltet intramolekular relativ leicht ein oder zwei Moleküle Wasser ab und bildet cyclische Ether. Sorbit läßt sich durch die Formel 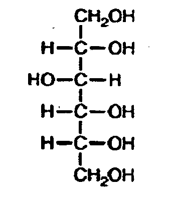 beschreiben und kommt in Form farbloser, mäßig hygroskopischer, optisch aktiver Nadeln, welche sich leicht in Wasser lösen, in den Handel.

Glycerin (1,2,3-Propantriol, 1,2,3-Trihydroxypropan, Glycerol, Ölsüß, INCI-Bezeichnung: Glycerin, E 422) ist eine farblose, klare, schwerbewegliche, geruchlose, süß schmeckende, hygroskopische Flüssigkeit, die mit Wasser und Alkohol in jedem Verhältnis mischbar ist.

### Glycerin läßt sich durch die Formel

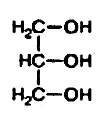

beschreiben. Die Herstellung von Glycerin erfolgte ursprünglich als Nebenprodukt der Fettverseifung. Die heutigen technischen Verfahren gehen von Propen aus, das über die Zwischenstufen Allylchlorid und Epichlorhydrin zu Glycerin verarbeitet wird. Ein weiteres technisches Verfahren ist die Hydroxylierung von Allylalkohol mit Wasserstoffperoxid am WO₃-Kontakt über die Stufe des Glycids.

1,2-Propylenglykol (1,2-Propandiol) ist eine farblose und stark hygroskopische Flüssigkeit, die in jedem Verhältnis mit Wasser und Alkoholen (wie Methanol, Ethanol, Propanolen, Butanolen) mischbar ist. Technisches 1,2-Propandiol ist ein Racemat aus (-)-(R)- und (+)-(S)-1,2-Prpylenglycol. Die Herstellung erfolgt über direkte Hydrolyse von Propylenoxid. Da 1,2-P. mit Propylenoxid weiterreagiert, entsteht dabei eine Mischung aus 1,2- und Tripropylenglykol, die durch Destillation getrennt werden muß. 1,2-Propylenglycol kann auch aus nachwachsenden Rohstoffen über drei unterschiedliche Routen hergestellt werden: a) Katalytische Hydrierung von Zuckern; b) Gärung von Zuckern zu Milchsäure und anschließend Hydrierung des Milchsäureesters; c) direkte Fermentation von Zuckern.

Für bestimmte Anwendungsbereiche kann es vorteilhaft sein, nur einen der drei oben genannten Inhaltsstoffe einzusetzen. In den meisten Fällen ist dabei Sorbit bevorzugt. Allerdings können auf anderen Anwendungsgebieten Mischungen von zwei der drei Stoffe oder aller drei Stoffe bevorzugt sein. Besonders vorteilhaft hat sich hier eine Mischung aus Glycerin, Sorbit und 1,2-Propylenglycol in einem Gewichtsverhältnis von 1 : (0,5-1) : (0,1-0,5) erwiesen.

Neben Sorbit bzw. Glycerin bzw. 1,2-Propylenglycol eignen sich als weitere mehrwertige Alkohole solche mit mindestens 2 OH-Gruppen, vorzugsweise Mannit, Xylitol, Polyethylenglycol, Polypropylenglycol und deren Mischungen.

Unter diesen Verbindungen sind diejenigen mit 2 bis 12 OH-Gruppen und insbesondere diejenigen mit 2, 3, 4, 5, 6 oder 10 OH-Gruppen bevorzugt.

Polyhydroxyverbindungen mit 2 OH-Gruppen sind beispielsweise Glycol (CH₂(OH)CH₂OH) und andere 1,2-Diole wie H-(CH₂)ₙ-CH(OH)CH₂OH mit n = 2, 3, 4, 5, 6, 7, 8, 9, 10, 11, 12, 13, 14, 15, 16, 17, 18, 19, 20. Auch 1,3-Diole wie H-(CH₂)ₙ-CH(OH) CH₂CH₂OH mit n = 1, 2, 3, 4, 5, 6, 7, 8, 9, 10, 11, 12, 13, 14, 15, 16, 17, 18, 19, 20 sind erfindungsgemäß einsetzbar. Die (n,n+1)- bzw. (n,n+2)-Diole mit nicht endständigen OH-Gruppen können ebenfalls eingesetzt werden.

Wichtige Vertreter von Polyhydroxyverbindungen mit 2 OH-Gruppen sind auch die Polyethylen- und Polypropylenglycole.

Als bevorzugte weitere mehrwertige Alkohole können z. B. Xylit, Propylenglycole, Polyethylenglycole, insbesondere solche mit mittleren Molekulargewichten von 200-800 eingesetzt werden.

Besonders bevorzugt ist der Einsatz von Sorbit, so daß Mittel, die außer Sorbit keine anderen mehrwertigen Alkohole enthalten, besonders bevorzugt sind.

Daneben ist auch der Einsatz von Glycerin bevorzugt, so daß auch Mittel, die außer Glycerin keine anderen mehrwertigen Alkohole enthalten, besonders bevorzugt sind.

Der Einsatz von Putzkörpern (Abrasiva) ist ebenfalls bevorzugt. Putzkörper sind amorphe, überwiegend anorganische, weitgehend wasserunlösliche, kleinstteilige Pulver, die keine scharfen Kanten aufweisen. Sie begünstigen in Zahn- und Mundpflegemitteln die Reinigung der Zähne und polieren gleichzeitig die Zahnoberfläche (Poliermittel).

Als Poliermittel eignen sich prinzipiell alle für Zahnpasten bekannten Reibkörper, insbesondere solche, die keine Calciumionen enthalten. Bevorzugt geeignete Poliermittel-komponenten sind daher Kieselsäuren, Aluminiumhydroxid, Aluminiumoxid, Natrium-aluminiumsilikate, organische Polymere oder Gemische solcher Reibkörper.

Calciumhaltige Polierkomponenten wie z.B. Kreide, Calciumpyrophosphat, Dicalcium-phosphatdihydrat können aber in Mengen bis zu 5 Gew.-% - bezogen auf die Gesamtzusammensetzung - enthalten sein.

Der Gesamtgehalt an Poliermitteln liegt vorzugsweise im Bereich von 5 - 50 Gew.-% des Zahnpflegemittels.

Besonders bevorzugt sind Zahnpasten und flüssige Zahnreinigungsmittel, die als Poliermittel Kieselsäuren enthalten. Geeignete Kieselsäuren sind z.B. Gelkieselsäuren, Hydrogelkieselsäuren und Fällungskieselsäuren. Gelkieselsäuren werden durch Umsetzung von Natriumsilikatlösungen mit starken, wäßrigen Mineralsäuren unter Ausbildung eines Hydrosols, Alterung zum Hydrogel, Waschen und Trocknen hergestellt. Erfolgt die Trocknung unter schonenden Bedingungen auf Wassergehalte von 15 bis 35 Gew.-%, so werden die so genannten Hydrogelkieselsäuren erhalten. Durch Trocknung auf Wassergehalte unterhalb 15 Gew.-% erfolgt eine irreversible Schrumpfung der vorher lockeren Struktur des Hydrogels zur dichten Struktur des sog. Xerogels.

Eine zweite, bevorzugt geeignete Gruppe von Kieselsäure-Poliermitteln sind die Fällungskieselsäuren. Diese werden durch Ausfällung von Kieselsäure aus verdünnten Alkalisilikat-Lösungen durch Zugabe von starken Säuren unter Bedingungen erhalten, bei welchen die Aggregation zum Sol und Gel nicht eintreten kann. Geeignete Verfahren zur Bevorzugt geeignet ist eine Fällungskieselsäure mit einer BET-Oberfläche von 15 - 110 m²/g, einer Partikelgröße von 0,5 - 20 µm, wobei wenigstens 80 Gew.-% der Primärpartikel unter 5 µm liegen sollen, und einer Viskosität in 30 %iger Glycerin-Wasser-(1 : 1)-Dispersion von 30 - 60 Pa.s (20°C) in einer Menge von 10 - 20 Gew.-% der Zahnpaste. Bevorzugt geeignete Fällungskieselsäuren dieser Art weisen außerdem gerundete Ecken und Kanten auf und sind unter der Handelsbezeichnung Sident^{®}12 DS (DEGUSSA) erhältlich.

Andere Fällungskieselsäuren dieser Art sind Sident^{®} 8 (DEGUSSA) und Sorbosil^{®} AC 39 (Crosfield Chemicals). Diese Kieselsäuren zeichnen sich durch eine geringere Verdickungswirkung und eine etwas höhere mittlere Teilchengröße von 8 -14 µm bei einer spezifischen Oberfläche von 40 - 75 m²/g (nach BET) aus und eignen sich besonders gut für flüssige Zahncremes. Diese sollten eine Viskosität (25°C, Scherrate D = 10 s⁻¹) von 10 - 100 Pa.s aufweisen.

Zahnpasten, die eine deutlich höhere Viskosität von mehr als 100 Pa.s (25° C, D = 10 s⁻¹) aufweisen, benötigen hingegen einen genügend hohen Anteil an Kieselsäuren mit einer Teilchengröße von weniger als 5 µm, bevorzugt wenigstens 3 Gew.-% einer Kieselsäure mit einer Partikelgröße von 1 - 3 µm. Solchen Zahnpasten setzt man daher bevorzugt neben den genannten Fällungskieselsäuren noch feinteiligere, so genannte Verdickungs-Kieselsäuren mit einer BET-Oberfläche von 150 -250 m²/g zu, z.B. die Handelsprodukte Sipernat 22 LS oder Sipernat^{®} 320 DS.

Als weitere Poliermittelkomponente kann auch z.B. Aluminiumoxid in Form von schwach calcinierter Tonerde mit einem Gehalt an - und -Aluminiumoxid in einer Menge von ca. 1 - 5 Gew.-% enthalten sein. Ein solches geeignetes Aluminiumoxid ist unter der Handelsbezeichnung "Poliertonerde P10 feinst" (Giulini Chemie) erhältlich.

Als Poliermittel eignen sich weiter alle für Zahnpasten bekannten Reibkörper wie z. B. Natriumaluminiumsilikate wie z. B. Zeolith A, organische Polymere wie z. B. Polymethacrylat oder Gemische dieser und der vorstehend genannten Reibkörper.

Zusammenfassend sind erfindungsgemäße Mund- und Zahnpflege- und -reinigungsmittel bevorzugt, die zusätzlich Putzkörper, vorzugsweise Kieselsäuren, Aluminiumhydroxid, Aluminiumoxid, Calciumpyrophosphat, Kreide, Dicalciumphosphat-dihydrat (CaHPO₄ 2H₂O), Natriumaluminiumsilikate, insbesondere Zeolith A, organische Polymere, insbesondere Polymethacrylate oder Gemische dieser Reibkörper,vorzugsweise in Mengen von 1 bis 30 Gew.%, vorzugsweise von 2,5 bis 25 Gew.% und insbesondere von 5 bis 22 Gew.%, jeweils bezogen auf das gesamte Mittel, enthalten.

Die angegebenen Mengen beziehen sich auf die Gesamtmenge an Reibkörpern, wobei einzelne Reibkörper vorzugsweise in engeren Mengenbereichen eingesetzt werden. Erfindungsgemäß bevorzugte Mittel enthalten beispielsweise 5 bis 20 Gew.-%, vorzugsweise 8 bis 21 Gew.-%, weiter bevorzugt 9 bis 20 Gew.-% und insbesondere 11 bis 19 Gew.-% Kieselsäure(n). Weiter bevorzugte erfindungsgemäße Mittel sind dadurch gekennzeichnet, daß sie 0,25 bis 2 Gew.-%, vorzugsweise 0,5 bis 1,5 Gew.-% und insbesondere 0,75 bis 1,25 Gew.-% Aluminiumoxid enthalten.

Mund- und Zahnpflege- und reinigungsmittel, insbesondere Zahnpasten, können z.B. auch Substanzen enthalten, die gegen Plaque und/oder Zahnstein wirksam sind.

Zahnbelag (Plaque) ist ein rauer, klebriger Belag auf den Zähnen, der aus Speichel, Bakterien und Nahrungsresten besteht. Setzen sich Mineralsalze (z.B. Calcium, Phosphat) aus dem Speichel im Zahnbelag ab, so bilden sich harte, weiße oder gelbliche Ablagerungen am Zahn, die man Zahnstein nennt. In dem porösen Zahnstein kann sich wiederum leicht Zahnbelag absetzen, der das Zahnfleisch angreift.

Die Bakterien auf der Zahnoberfläche bauen Kohlenhydrate, besonders Zucker, aus der Nahrung zu Säure ab. Diese Säure löst die Zahnsubstanz auf und es kommt zu Karies (Zahnfäule). Dabei werden besonders die Mineralien Calcium und Phosphat aus dem Zahnschmelz herausgelöst. Nach dem Zahnschmelzmantel werden auch innere Schichten des Zahnes angegriffen. Bakterien können in das Zahnmark eindringen und dort zu Entzündungen führen. Meist kommt es dann zu stechenden Zahnschmerzen.

Wie bereits erwähnt, beinhaltet Plaque Bakterien, so daß sich zur Bekämpfung von Plaque antimikrobielle Stoffe eignen. Diese besitzen darüber hinaus eine Wirkung als Konservierungsmittel.

In Mund und Zahnpflege- und -reinigungsmitteln können beispielsweise die auch in Lebensmitteln zugelassenen Konservierungsmittel Sorbinsäure (E 200), Kaliumsorbat (E 202), Calciumsorbat (E 203), Benzoesäure (E 210), Natriumbenzoat (E 211), Kaliumbenzoat (E 212), Calciumbenzoat (E 213), Ethyl-4-hydroxybenzoat (E 214), Ethyl-4-hydroxybenzoat, Natriumsalz (E 215), Propyl-4-hydroxybenzoat (E 216), Propyl-4-hydroxybenzoat, Natriumsalz (E 217), Methyl-4-hydroxybenzoat (E 218), Methyl-4-hydroxybenzoat, Natriumsalz (E 219), Schwefeldioxid (schweflige Säure), (E 220), Natriumsulfit (E 221), Natriumhydrogensulfit (E 222), Natriumdisulfit (E 223), Kaliumdisulfit (E 224), Calciumsulfit (E 226), Calciumhydrogensulfit (E 227), Kaliumhydrogensulfit (E 228), Biphenyl (E 230), Orthophenylphenol (2-Biphenylol), (E 231), Natriumorthophenylphenolat (E 232), Nisin (E 234), Natamycin (E 235), Ameisensäure (E 236), Natriumformiat (E 237), Calciumformiat (E 238), Hexamethylentetramin (E 239), Dimethyldicarbonat (E 242), Kaliumnitrit (E 249), Natriumnitrit (E 250), Natriumnitrat (E 251), Essigsäure (E 260), Kaliumacetat (E 261), Natriumacetat (E 262), Calciumacetat (E 263), Milchsäure (E 270), Propionsäure (E 280), Natriumpropionat (E 281), Calciumpropionat (E 282), Kaliumpropionat (E 283), Borsäure (E 284), Natriumtetraborat (E 285), Hydroxybernsteinsäure (Äpfelsäure), (E 296), Fumarsäure (E 297), Lysozym (E 1105), eingesetzt werden.

Bevorzugte Stoffe sind ausgewählt aus p-Hydroxybenzoesäuremethyl-, -ethyl- oder propylester, Natriumsorbat, Natriumbenzoat, Bromchlorophen, Triclosan, Phenyl- Salicylsäureester, Biguaniden z. B. Chlorhexidin (1,1'-Hexamethylenbis[5-(4-chlorphenyl)-biguanid), Thymol usw..

Erfindungsgemäß bevorzugte Mund- und Zahnpflege- und -reinigungsmittel sind dadurch gekennzeichnet, daß sie zusätzlich Antiplaque-Wirkstoffe, vorzugsweise p- Hydroxybenzoesäuremethyl-, -ethyl- oder propylester, Natriumsorbat, Natriumbenzoat, Bromchlorophen, Triclosan, Hexetidine, Phenyl- Salicylsäureester, Biguanide z. B. Chlorhexidin, Thymol, vorzugsweise in Mengen von 0,05 bis 5 Gew.%, vorzugsweise von 0,10 bis 2,5 Gew.% und insbesondere von 0,30 bis 1,5 Gew.%, jeweils bezogen auf das gesamte Mittel, enthalten.

Die angegebenen Mengen beziehen sich auf die Gesamtmenge an Antiplaque-Wirkstoffen, wobei einzelne Wirkstoffe vorzugsweise in engeren Mengenbereichen eingesetzt werden. Erfindungsgemäß bevorzugte Mittel enthalten beispielsweise 0,1 bis 2 Gew.-%, vorzugsweise 0,2 bis 1,75 Gew.-%, weiter bevorzugt 0,3 bis 1,5 Gew.-% und insbesondere 0,4 bis 1,0 Gew.-% Natriumbenzoat. Weiter bevorzugte erfindungsgemäße Mittel sind dadurch gekennzeichnet, daß sie 0,005 bis 0,1 Gew.-%, vorzugsweise 0,01 bis 0,075 Gew.-% und insbesondere 0,02 bis 0,05 Gew.-% Chlorhexidin enthalten. Chlorhexidin wird vorzugsweise gemeinsam mit Alkylpolyglycosiden (APG) eingesetzt, wobei in bevorzugten erfindungsgemäßen Mitteln als Alkylglykoside solche mit 8-18 C-Atomen in der Alkylgruppe und einem mittleren Oligomerisationsgrad des Glycosidrestes von 1-3 in einer Menge von 0,025 bis 2,5 Gew.-% enthalten sind.

Gegen Zahnstein wirksame Stoffe können beispielsweise Chelatbildner sein wie z. B. Ethylendiamintetraessigsäure und deren Natriumsalze, Pyrophosphat- Salze wie die wasserlöslichen Dialkali- oder Tetraalkalimetallpyrophosphat- Salze, z. B. Na₄P₂O₇, K₄P ₂O₇, Na₂K₂P₂O ₇, Na₂H₂P₂O₇ und K ₂H₂P₂O₇ oder Polyphosphat-Salze, die z. B. aus wasserlöslichen Alkalimethalltripolyphosphaten wie Natriumtripolyphosphat und Kaliumtripolyphosphat ausgewählt sein können.

Erfindungsgemäß bevorzugte Mund- und Zahnpflege- und -reinigungsmittel sind dadurch gekennzeichnet, daß sie zusätzlich Phosphat(e), vorzugsweise Alkalimetallphosphat(e) und insbesondere Natriumtripolyphosphat, vorzugsweise in Mengen von 1 bis 10 Gew.-%, besonders bevorzugt von 2 bis 8 Gew.-% und insbesondere von 3 bis 7 Gew.-%, jeweils bezogen auf das gesamte Mittel, enthalten.

Als Konsistenzregler (bzw. Bindemittel) dienen z. B. natürliche und/oder synthetische wasserlösliche Polymere wie Alginate, Carragheenate, Traganth, Stärke und Stärkeether, Celluloseether wie z. B. Carboxymethylcellulose (Na-Salz), Hydroxyethylcellulose, Methylhydroxypropylcellulose, Guar, Akaziengum, Agar-Agar, Xanthan- Gum, Succinoglycan-Gum, Johannisbrotmehl, Pectine, wasserlösliche Carboxyvinylpolymere (z. B. Carbopol®-Typen), Polyvinylalkohol, Polyvinylpyrrolidon, Polyethylenglycole, insbesondere solche mit Molekulargewichten von 1 500-1 000 000.

Weitere Stoffe, die sich zur Viskositätskontrolle eignen, sind z. B. Schichtsilikate wie z. B. Montmorillonit-Tone, kolloidale Verdickungskieselsäuren wie z. B. Aerogel-Kieselsäuren, pyrogene Kieselsäuren oder feinstvermahlene Fällungskieselsäuren. Es können auch viskositätsstabilisierende Zusätze aus der Gruppe der kationischen, zwitterionischen oder ampholytischen stickstoffhaltigen Tenside, der hydroxypropylsubstituierten Hydrocolloide oder der Polyethylenglycol/Polypropylenglycol- Copolymere mit einem mittleren Molgewicht von 1000 bis 5000 oder eine Kombination der genannten Verbindungen in den Zahnpasten verwendet werden.

Auch oberflächenaktive Substanzen sind in den Zahnpasten zur Unterstützung der Reinigungswirkung und gewünschtenfalls auch zur Entwicklung von Schaum beim Zähnebürsten sowie zur Stabilisierung der Polierkörperdispersion im Träger in einer Menge von 0,1-5 Gew.-% enthalten.

Geeignete Tenside sind z. B. lineare Natriumalkylsulfate mit 12-18 C-Atomen in der Alkylgruppe. Diese Stoffe weisen zusätzlich eine enzymhemmende Wirkung auf den bakteriellen Stoffwechsel des Zahnbelags auf. Weitere geeignete Tenside sind Alkalisalze, bevorzugt Natriumsalze von Alkylpolyglycolethersulfat mit 12-16 C-Atomen in der linearen Alkylgruppe und 2-6 Glycolethergruppen im Molekül, von linearem Alkan(C₁₂- C₁₈)-sulfonat, von Sulfobernsteinsäuremonoalkyl(C₁₂-C₁₈)-estern, von sulfatisierten Fettsäuremonoglyceriden, sulfatisierten Fettsäurealkanolamiden, Sulfoessigsäurealkyl(C₁₂-C₁₆ )-estern, Acylsarcosinen, Acyltauriden und Acylisothionaten mit jeweils 8-18 C-Atomen in der Acylgruppe. Auch zwitterionische, ampholytische und nichtionische Tenside sind geeignet, z. B. Oxethylate von Fettsäuremono- und -diglyceriden, von Fettsäure-Sorbitanestern und Alkyl(oligo)-Glucoside.

Die erfindungsgemäßen Mittel, insbesondere die Zahnpasten, können auch Substanzen zur Erhöhung des mineralisierenden Potentials enthalten, beispielsweise calciumhaltige Substanzen wie z. B. Calciumchlorid, Calciumacetat und Dicalciumphosphat-Dihydrat. Die Konzentration der calciumhaltigen Substanz hängt von der Löslichkeit der Substanz und dem Zusammenwirken mit anderen in dem Mund- und Zahnpflegemittel enthaltenen Substanzen ab.

Mund und Zahnpflege- und -reinigungsmittel können darüber hinaus mit besonderem Vorzug Antikaries-Wirkstoffe enthalten. Diese können beispielsweise aus organischen oder anorganischen Fluoriden ausgewählt sein, z. B. aus Natriumfluorid, Kaliumfluorid, Natriummonofluorphosphat und Natriumfluorosilikat. Auch Zinkfluorid, Zinn-(II)-fluorid sind bevorzugt. Bevorzugt sollte eine Menge von 0,01 - 0,2 Gew.-% Fluor in Form der genannten Verbindungen enthalten sein.

Erfindungsgemäße Mund- und Zahnpflege- und -reinigungsmittel, die zusätzlich Antikaries-Wirkstoffe, vorzugsweise Fluorverbindung(en), insbesondere Natriumfluorid, Kaliumfluorid, Natriummonofluorphosphat, Zinkfluorid, Zinnfluorid und Natriumfluorosilikat, vorzugsweise in Mengen von 0,01 bis 5 Gew.%, vorzugsweise von 0,1 bis 2,5 Gew.% und insbesondere von 0,2 bis 1,1 Gew.%, jeweils bezogen auf das gesamte Mittel, enthalten, sind erfindungsgemäß bevorzugt.

Die erfindungsgemäßen Zahnpflegemittel können z.B. durch Zusatz von Aromaölen und Süßungsmitteln in ihren organoleptischen Eigenschaften verbessert werden.

Als Aromaöle können alle die für Mund- und Zahnpflegemittel üblichen natürlichen und synthetischen Aromen eingesetzt werden. Natürliche Aromen können sowohl in Form der aus Drogen isolierten natürlichen ätherischen Öle als auch der daraus isolierten Einzelkomponenten enthalten sein.

Geeignete Aromen sind z.B. Pfefferminzöl, Krauseminzöl, Eukalyptusöl, Anisöl, Fenchelöl, Kümmelöl, Menthylacetat, Zimtaldehyd, Anethol, Vanillin, Thymol sowie Mischungen dieser Komponenten.

Geeignete Süßungsmittel sind z.B. Saccharin-Natrium, Natrium-Cyclamat, Sucrose, Lactose, Meltose, Fructose.

Weitere übliche Hilfs- und Zusatzstoffe für Zahnpasten sind
- Oberflächenaktive Stoffe, bevorzugt anionische, zwitterionische, amphotere, nichtionische Tenside oder eine Kombination mehrerer verschiedener Tenside
- Lösungsmittel und Lösungsvermittler, z.B. niedere einwertige oder mehrwertige Alkohole oder Ether, z.B. Ethanol, 1,2-Propylenglycol, Diethylenglycol oder Butyldi- glycol
- Pigmente, wie z.B. Titandioxid
- Farbstoffe
- Puffersubstanzen, z.B. primäre, sekundäre oder tertiäre Alkaliphosphate oder Citronensäure-/Na-Citrat
- weitere wundheilende oder entzündungshemmende Stoffe, z.B. Allantoin, Harnstoff, Azulen, Kamillewirkstoffe, Acetylsalicylsäurederivate oder Rhodanid
- weitere Vitamine wie z.B. Ascorbinsäure, Biotin, Tocopherol oder Rutin
- Mineralsalze wie z.B. Mangan-, Zink- oder Magnesiumsalze.
Eine weitere wichtige Gruppe von Inhaltstoffen, die in den erfindungsgemäßen Mitteln enthalten sein kann, sind die so genannten bioaktiven Gläser.
Der Begriff "bioaktive Gläser" umfaßt im Rahmen der vorliegenden Anmeldung Gläser, welche biologisch wirksam und/oder biologisch aktiv sind. Die biologische Wirksamkeit eines Glases kann sich beispielsweise in dessen antimikrobiellen Eigenschaften zeigen, biologisch aktives Glas unterscheidet sich von herkömmlichen Kalk-Natrium-Silicat-Gläsern dadurch, daß es lebendes Gewebe bindet. Biologisch aktives Glas bezeichnet dabei beispielsweise ein Glas, das eine feste Bindung mit Körpergewebe eingeht, wobei eine Hydroxyl-Apatitschicht ausgebildet wird. Unter bioaktivem Glas wird auch ein Glas verstanden, das antimikrobielle und/oder entzündungshemmende Wirkung zeigt. Die Glaspulver zeigen gegenüber Bakterien, Pilzen sowie Viren eine biozide bzw. eine biostatische Wirkung; sind im Kontakt mit dem Menschen hautverträglich, toxikologisch unbedenklich und insbesondere auch zum Verzehr geeignet.
Erfindungsgemäß besonders bevorzugte Mund- und Zahnpflege- und -reinigungsmittel sind dadurch gekennzeichnet, daß sie 0,2 bis 20 Gew.-%, vorzugsweise 0,4 bis 14 Gew.-%, besonders bevorzugt 0,5 bis 3 Gew.-% und insbesondere 0,6 bis 2 Gew.-% mindestens eines bioaktiven Glases enthalten.
Die erfindungsgemäßen Mund- und Zahnpflege- und -reinigungsmittel dieser Ausführungsform enthalten bioaktives Glas oder Glaspulver oder Glaskeramikpulver oder Kompositmaterialien, welche ein solches bioaktives Glas umfassen. Unter Glaspulvern werden im Rahmen der vorliegenden Anmeldung auch Granulate und Glaskügelchen verstanden.
Aufgrund der Anforderungen an die toxikologische Unbedenklichkeit des Glases sowie deren Eignung zum Verzehr soll das Glaspulver besonders rein sein. Die Belastung durch Schwermetalle ist vorzugsweise gering. So beträgt die Maximalkonzentration im Bereich der kosmetischen Formulierungen vorzugsweise für Pb < 20 ppm, Cd < 5 ppm, As < 5 ppm, Sb < 10 ppm, Hg < 1 ppm, Ni < 10 ppm.
Das unkeramisierte Ausgangsglas, das direkt in den bevorzugten erfindungsgemäßen Zusammensetzungen enthalten oder gegebenenfalls für die Herstellung einer erfindungsgemäß einsetzbaren Glaskeramik verwandt wird, enthält SiO₂ als Netzwerkbildner, vorzugsweise zwischen 35-80 Gew.-%. Bei niedrigeren Konzentrationen nimmt die spontane Kristallisationsneigung stark zu und die chemische Beständigkeit stark ab. Bei höheren SiO₂-Werten kann die Kristallisationsstabilität abnehmen, und die Verarbeitungstemperatur wird deutlich erhöht, so daß sich die Heißformgebungseigenschaften verschlechtern. Na₂O wird als Flußmittel beim Schmelzen des Glases eingesetzt. Bei Konzentrationen kleiner 5% wird das Schmelzverhalten negativ beeinflußt. Natrium ist Bestandteil der sich bei der Keramisierung bildenden Phasen und muß, sofern hohe kristalline Phasenanteile durch die Keramisierung eingestellt werden sollen, in entsprechend hohen Konzentrationen im Glas enthalten sein. K₂O wirkt als Flußmittel beim Schmelzen des Glases. Außerdem wird Kalium in wässrigen Systemen abgegeben. Liegen hohe Kaliumkonzentrationen im Glas vor, werden kaliumhaltige Phasen wie Kalzium-Silicaten ebenfalls ausgeschieden. Über den P₂O₅-Gehalt kann bei silikatischen Gläsern, Glaskeramiken oder Kompositen die chemische Beständigkeit des Glases und damit die lonenabgabe in wässrigen Medien eingestellt werden. Bei Phospahtgläsern ist P₂O₅ Netzwerkbilder. Der P₂O₅-Gehalt liegt vorzugsweise zwischen 0 und 80 Gew.-%. Um die Schmelzbarkeit zu verbessern, kann das Glas bis zu 25 Gew.- % B₂O₃ enthalten. Al₂O₃ wird genutzt, um die chemische Beständigkeit des Glases einzustellen.
Zur Verstärkung der antimikrobiellen, insbesondere der antibakteriellen Eigenschaften der Glaskeramik können antimikrobiell wirkende Ionen wie z. B. Ag, Au, I, Ce, Cu, Zn in Konzentrationen kleiner 5 Gew.-% enthalten sein.
Farbgebende Ionen wie z. B. Mn, Cu, Fe, Cr, Co, V, können einzeln oder kombiniert, vorzugsweise in einer Gesamtkonzentration kleiner 1 Gew.-%, enthalten sein.
Üblicherweise wird das Glas bzw. die Glaskeramik in Pulverform eingesetzt. Die Keramisierung kann entweder mit einem Glasblock bzw. Glasribbons erfolgen oder aber mit Glaspulver. Nach der Keramisierung müssen die Glaskeramikblöcke oder Ribbons zu Pulver gemahlen werden. Wurde das Pulver keramisiert, muß gegebenenfalls auch erneut gemahlen werden, um Agglomerate, die während des Keramisierungsschrittes entständen sind, zu entfernen. Die Mahlungen können sowohl trocken als auch in wässrigen oder nicht wässrigen Mahlmedien durchgeführt werden. Üblicherweise liegen die Partikelgrößen kleiner 500 µm. Als zweckmäßig haben sich Partikelgrößen < 100 µm bzw. < 20 µm erwiesen. Besonders geeignet sind Partikelgrößen < 10 µm sowie kleiner 5 µm sowie kleiner 2 µm, siehe weiter unten.
Die in den bevorzugten erfindungsgemäßen Zusammensetzungen enthaltenen bioaktiven Gläser bzw. Glaspulver oder Glaskeramikpulver oder Komposit-Zusammensetzungen umfassen Gläser, die bevorzugt nachfolgende Komponenten umfassen: SiO₂: 35-80 Gew.-%, Na₂O: 0-35 Gew.-%, P₂O₅: 0-80 Gew.-%, MgO: 0-5 Gew.-%, Ag₂O: 0-0,5 Gew.-%, AgJ: 0-0,5 Gew.- %, NaJ: 0-5 Gew.-%, TiO₂: 0-5 Gew.-%, K₂O: 0-35 Gew.-%, ZnO: 0-10 Gew.-%, Al ₂O₃: 0-25 Gew.-% undB₂O₃: 0-25 Gew.-%.
Weiterhin können dem Grundglas gemäß obiger Zusammensetzung zur Erzielung weiterer Effekte wie beispielsweise Farbigkeit oder UV-Filterung Ionen wie Fe, Co, Cr, V, Ce, Cu, Mn, Ni, Bi, Sn, Ag, Au, J einzeln oder in Summe bis zu 10 Gew.-% zugegeben werden. Eine weiter Glaszusammensetzung kann wie folgt sein: SiO ₂: 35-80 Gew.-%, Na₂O: 0-35 Gew.-%, P₂O₅: 0-80 Gew.-%, MgO: 0-5 Gew.- %, Ag₂O: 0-0,5 Gew.-%, AgJ: 0-0,5 Gew.-%, NaJ: 0-5 Gew.-%, TiO₂: 0-5 Gew.-%, K₂O: 0-35 Gew.-%, ZnO: 0-10 Gew.-%, Al₂ O₃: 0-25 Gew.-%, B₂O₃: 0- 25 Gew.-%, SnO: 0-5 Gew.-%, CeO₂: 0-3 Gew.- % und Au: 0,001-0,1 Gew.-%.
Besonders bevorzugte erfindungsgemäße Mund- und Zahnpflege- und -reinigungsmittel sind dadurch gekennzeichnet, daß das bioaktive Glas - bezogen auf sein Gewicht - folgende Zusammensetzung aufweist:

| | | | |
|---|---|---|---|
| SiO₂ | 35 bis 60 Gew.-%, | vorzugsweise | 40 bis 60 Gew.-%, |
| Na₂O | 0 bis 35 Gew.-%, | vorzugsweise | 5 bis 30 Gew.-%, |
| K₂O | 0 bis 35 Gew.-%, | vorzugsweise | 0 bis 20 Gew.-%, |
| P₂O₅ | 0 bis 10 Gew.-%, | vorzugsweise | 2 bis 10 Gew.-%, |
| MgO | 0 bis 10 Gew.-%, | vorzugsweise | 0 bis 5 Gew.-%, |
| CaO | 0 bis 35 Gew.-%, | vorzugsweise | 5 bis 30 Gew.-%, |
| Al₂O₃ | 0 bis 25 Gew.-%, | vorzugsweise | 0 bis 5 Gew.-%, |
| B₂O₃ | 0 bis 25 Gew.-%, | vorzugsweise | 0 bis 5 Gew.-%, |
| TiO₂ | 0 bis 10 Gew.-%, | vorzugsweise | 0,1 bis 5 Gew.-%. |

Wie bereits weiter oben erwähnt, wird das bioaktive Glas vorzugsweise in partikulärer Form eingesetzt. Hier sind besonders bevorzugte erfindungsgemäße Mund- und Zahnpflege- und - reinigungsmittel dadurch gekennzeichnet, daß das antimikrobielle Glas Teilchengrößen < 10 µm, vorzugsweise von 0,5 bis 4 µm, besonders bevorzugt von 1 bis 2 µm, aufweist.
Es hat sich gezeigt, daß die erfindungsgemäßen Mund- und Zahnpflege- und -reinigungsmittel in ihrer Leistung weiter gesteigert werden können, wenn die Mittel salivationsfördernde Substanzen enthalten. Insbesondere die antibakterielle Wirkung und mit ihr die Antikarieswirkung und die Wirkung gegen Gingivitis und/oder Parodontitis werden hierdurch verstärkt.
Unter Salivation versteht man die Speichelproduktion und -freisetzung, im weiteren Sinne auch in unphysiologisch erhöhter Menge. Substanzen, die den Speichelfluß anregen und die Speichelmenge und/oder-freisetzung erhöhen, können aus den unterschiedlichsten Stoffklassen stammen.
Eine erfindungsgemäß beispielsweise geeignete Substanz ist das Pilocarpin, das in den erfindungsgemäßen Mund- und Zahnpflege- und -reinigungsmitteln enthalten sein kann. 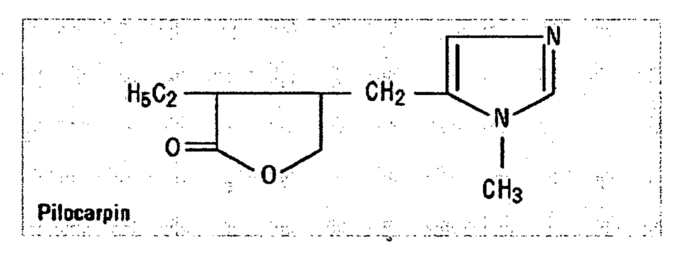 Weitere salivationsfördernde Substanzen sind insbesondere so genannte Scharfstoffe, d.h. scharf schmeckende und/oder ein Gefühl von Wärme erzeugende Substanzen. Erfindungsgemäß bevorzugte Mund- und Zahnpflege- und -reinigungsmittel sind dadurch gekennzeichnet, daß sie als salivationsfördernde Substanz mindestens eine scharf schmeckende und/oder ein Gefühl von Wärme erzeugende Substanz enthalten.
Als salivationsfördernden Inhaltsstoff enthalten die erfindungsgemäßen Erzeugnisse dieser Ausführungsform eine scharf schmeckende und/oder ein Gefühl von Wärme erzeugende Substanz. Diese Substanzen vermitteln dem Anwender einen scharfen, kribbelnden, mundwässernden oder wärmeerzeugenden Effekt, d.h. sie rufen sensorisch einen Wärmeeindruck oder ein Brennen, oder ein Prickeln, Perlen, Kitzeln oder Sprudeln hervor und fördern dadurch den Speichelfluß.
Erfindungsgemäß bevorzugte Erzeugnisse dieser Ausführungsform enthalten die scharf schmeckende(n) und/oder ein Gefühl von Wärme erzeugende(n) Substanz(en) in Mengen von 0,00001 bis 5 Gew.-%, vorzugsweise von 0,0005 bis 2,5 Gew.-%, weiter bevorzugt von 0,001 bis 1 Gew.-%, besonders bevorzugt von 0,005 bis 0,75 Gew.-% und insbesondere von 0,01 bis 0,5 Gew.-%, jeweils bezogen auf das Gewichts des gesamten Mittels.
Als scharf schmeckende oder ein Gefühl von Wärme erzeugende Substanz kann eine Reihe von Stoffen eingesetzt werden. Bevorzugt sind insbesondere N-Alkyl-substituierte Amide von ungesättigten Carbonsäuren, beispielsweise
- 2E,4E-Decadiensäure-N-Methylamid
- 2E,4E-Decadiensäure-N-Ethylamid
- 2E,4E-Decadiensäure-N-n-Propylamid
- 2E,4E-Decadiensäure-N-Isopropylamid
- 2E,4E-Decadiensäure-N-n-Butylamid
- 2E,4E-Decadiensäure-N-(1-Methylpropyl)-amid
- 2E,4E-Decadiensäure-N-Isobutylamid
- 2E,4E-Decadiensäure-N-tert-Butylamid
- 2E,4Z-Decadiensäure-N-Methylamid
- 2E,4Z-Decadiensäure-N-Ethylamid
- 2E,4Z-Decadiensäure-N-n-Propylamid
- 2E,4Z-Decadiensäure-N-Isopropylamid
- 2E,4Z-Decadiensäure-N-n-Butylamid
- 2E,4Z-Decadiensäure-N-(1-Methylpropyl)-amid
- 2E,4Z-Decadiensäure-N-Isobutylamid
- 2E,4Z-Decadiensäure-N-tert-Butylamid
- 2E,4E,8Z-Decatriensäure-N-Methylamid
- 2E,4E,8Z-Decatriensäure-N-Ethylamid
- 2E,4E,8Z-Decatriensäure-N-n-Propylamid
- 2E,4E,8Z-Decatriensäure-N-Isopropylamid
- 2E,4E,8Z-Decatriensäure-N-n-Butylamid
- 2E,4E,8Z-Decatriensäure-N-(1-Methylpropyl)-amid
- 2E,4E,8Z-Decatriensäure-N-Isobutylamid
- 2E,4E,8Z-Decatriensäure-N-tert-Butylamid
- 2E,4Z,8Z-Decatriensäure-N-Methylamid
- 2E,4Z,8Z-Decatriensäure-N-Ethylamid
- 2E,4Z,8Z-Decatriensäure-N-n-Propylamid
- 2E,4Z,8Z-Decatriensäure-N-Isopropylamid
- 2E,4Z,8Z-Decatriensäure-N-n-Butylamid
- 2E,4Z,8Z-Decatriensäure-N-(1-Methylpropyl)-amid
- 2E,4Z,8Z-Decatriensäure-N-1sobutylamid
- 2E,4Z,8Z-Decatriensäure-N-tert-Butylamid
- 2E,4E,8E-Decatriensäure-N-Methylamid
- 2E,4E,8E-Decatriensäure-N-Ethylamid
- 2E,4E,8E-Decatriensäure-N-n-Propylamid
- 2E,4E,8E-Decatriensäure-N-Isopropylamid
- 2E,4E,8E-Decatriensäure-N-n-Butylamid
- 2E,4E,8E-Decatriensäure-N-(1-Methylpropyl)-amid
- 2E,4E,8E-Decatriensäure-N-Isobutylamid
- 2E,4E,8E-Decatriensäure-N-tert-Butylamid
- 2E,4Z,8E-Decatriensäure-N-Methylamid
- 2E,4Z,8E-Decatriensäure-N-Ethylamid
- 2E,4Z,8E-Decatriensäure-N-n-Propylamid
- 2E,4Z,8E-Decatriensäure-N-Isopropylamid
- 2E,4Z,8E-Decatriensäure-N-n-Butylamid
- 2E,4Z,8E-Decatriensäure-N-(1-Methylpropyl)-amid
- 2E,4Z,8E-Decatriensäure-N-1sobutylamid
- 2E,4Z,8E-Decatriensäure-N-tert-Butylamid
- 2E,6Z,8E-Decatriensäure-N-Methylamid
- 2E,6Z,8E-Decatriensäure-N-Ethylamid
- 2E,6Z,8E-Decatriensäure-N-n-Propylamid
- 2E,6Z,8E-Decatriensäure-N-Isopropylamid
- 2E,6Z,8E-Decatriensäure-N-n-Butylamid
- 2E,6Z,8E-Decatriensäure-N-(1-Methylpropyl)-amid
- 2E,6Z,8E-Decatriensäure-N-Isobutylamid
- 2E,6Z,8E-Decatriensäure-N-tert-Butylamid
- 2E,6E,8E-Decatriensäure-N-Methylamid
- 2E,6E,8E-Decatriensäure-N-Ethylamid
- 2E,6E,8E-Decatriensäure-N-n-Propylamid
- 2E,6E,8E-Decatriensäure-N-Isopropylamid
- 2E,6E,8E-Decatriensäure-N-n-Butylamid
- 2E,6E,8E-Decatriensäure-N-(1-Methylpropyl)-amid
- 2E,6E,8E-Decatriensäure-N-Isobutylamid
- 2E,6E,8E-Decatriensäure-N-tert-Butylamid
- 2E,6Z,8Z-Decatriensäure-N-Methylamid
- 2E,6Z,8Z-Decatriensäure-N-Ethylamid
- 2E,6Z,8Z-Decatriensäure-N-n-Propylamid
- 2E,6Z,8Z-Decatriensäure-N-Isopropylamid
- 2E,6Z,8Z-Decatriensäure-N-n-Butylamid
- 2E,6Z,8Z-Decatriensäure-N-(1-Methylpropyl)-amid
- 2E,6Z,8Z-Decatriensäure-N-Isobutylamid
- 2E,6Z,8Z-Decatriensäure-N-tert-Butylamid
- 2E,6E,8Z-Decatriensäure-N-Methylamid
- 2E,6E,8Z-Decatriensäure-N-Ethylamid
- 2E,6E,8Z-Decatriensäure-N-n-Propylamid
- 2E,6E,8Z-Decatriensäure-N-Isopropylamid
- 2E,6E,8Z-Decatriensäure-N-n-Butylamid
- 2E,6E,8Z-Decatriensäure-N-(1-Methylpropyl)-amid
- 2E,6E,8Z-Decatriensäure-N-Isobutylamid
- 2E,6E,8Z-Decatriensäure-N-tert-Butylamid
- 2E,7Z,9E-Undecatriensäure -N-Methylamid
- 2E,7Z,9E-Undecatriensäure -N-Ethylamid
- 2E,7Z,9E-Undecatriensäure -N-n-Propylamid
- 2E,7Z,9E-Undecatriensäure -N-Isopropylamid
- 2E,7Z,9E-Undecatriensäure -N-n-Butylamid
- 2E,7Z,9E-Undecatriensäure -N-(1-Methylpropyl)-amid
- 2E,7Z,9E-Undecatriensäure-N-isobutylamid
- 2E,7Z,9E-Undecatriensäure -N-tert-Butylamid
- 2E,7E,9E-Undecatriensäure -N-Methylamid
- 2E,7E,9E-Undecatriensäure -N-Ethylamid
- 2E,7E,9E-Undecatriensäure -N-n-Propylamid
- 2E,7E,9E-Undecatriensäure -N-Isopropylamid
- 2E,7E,9E-Undecatriensäure -N-n-Butylamid
- 2E,7E,9E-Undecatriensäure -N-(1-Methylpropyl)-amid
- 2E,7E,9E-Undecatriensäure-N-isobutylamid
- 2E,7E,9E-Undecatriensäure -N-tert-Butylamid
- 2E,7Z,9Z-Undecatriensäure -N-Methylamid
- 2E,7Z,9Z-Undecatriensäure -N-Ethylamid
- 2E,7Z,9Z-Undecatriensäure -N-n-Propylamid
- 2E,7Z,9Z-Undecatriensäure -N-Isopropylamid
- 2E,7Z,9Z-Undecatriensäure -N-n-Butylamid
- 2E,7Z,9Z-Undecatriensäure -N-(1-Methylpropyl)-amid
- 2E,7Z,9Z-Undecatriensäure-N-isobutylamid
- 2E,7Z,9Z-Undecatriensäure -N-tert-Butylamid
- 2E,7Z,9E-Undecatriensäure -N-Methylamid
- 2E,7Z,9E-Undecatriensäure -N-Ethylamid
- 2E,7Z,9E-Undecatriensäure -N-n-Propylamid
- 2E,7Z,9E-Undecatriensäure -N-Isopropylamid
- 2E,7Z,9E-Undecatriensäure -N-n-Butylamid
- 2E,7Z,9E-Undecatriensäure -N-(1-Methylpropyl)-amid
- 2E,7Z,9E-Undecatriensäure-N-isobutylamid
- 2E,7Z,9E-Undecatriensäure -N-tert-Butylamid

Selbstverständlich sind auch andere Substitutionsmuster am Stickstoffatom möglich und bevorzugt, beispielsweise längerkettige n-Alkylreste (...-N-n-Pentylamid, ...-N-n-Pentylamid, ...-N-n-Pentylamid, ...-N-n-Pentylamid, ...-N-n-Pentylamid, ...-N-n-Hexylamid, ...-N-n-Heptylamid, ...-N-n-Octylamid, ...-N-n-Nonylamid, ...-N-n-Decylamid, ...-N-n-Undecylamid, ...-N-n-Dodecylamid, ...-N-n-Tridecylamid, usw.) oder disubstituierte ...-N,N-Dialkylamide wie ...-N,N-dimethylamid, ...-N,N-diethylamid, ...-N,N-di-n-propylamid, ...-N,N-diisopropylamid, ...-N,N-di-n-butylamid, ...-N,N-di(1-Methylpropyl)amid, ...-N,N-diisobutylamid, ...-N,N-di-tert-butylamid, ...-N,N-methyl-ethylamid,...-N,N-methyl-n-propylamid, ...-N,N-methyl-isopropylamid, ...-N,N-ethyl-n-propylamid, ...-N,N-ethyl-isopropylamid, usw..

Unter den genannten Verbindungen sind einige im Rahmen der vorliegenden Erfindung besonders bevorzugt. Diese sind nachfolgend aufgeführt:
2E,6Z,8E-Decatriensäure-N-isobutylamid (N-Isobutyl-2E,6Z,8E-decatrienamid, auch Spilanthol oder Affinin genannt): 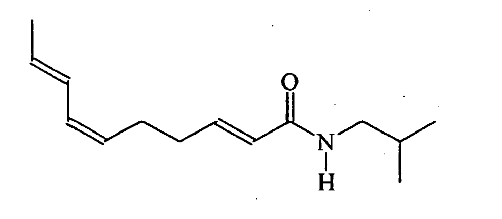 2E,4E,8Z-Decatriensäure-N-isobutylamid (N-Isobutyl-2E,4E,8Z-decatrienamid, auch Spilanthol oder Affinin genannt): 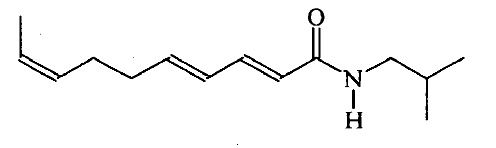 2E,7Z,9E-Undecatriensäure-N-isobutylamid (N-Isobutyl-2E,7Z,9E-undecatrienamid,): 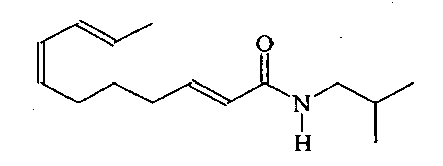 2E,4Z-Decatriensäure-N-isobutylamid (cis- Pellitrorin): 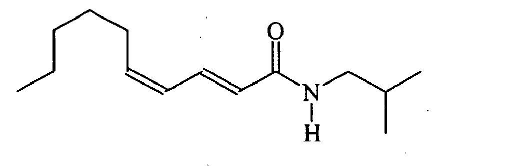 2E, 4-Decatriensäure-N-isobutylamid (trans- Pellitrorin): 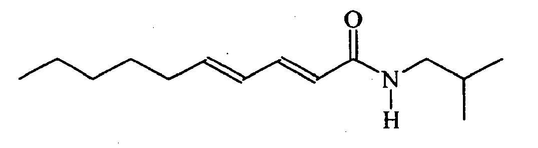 Ferulasäureamide, beispielsweise Ferulasäure-N-Vanillylamid: 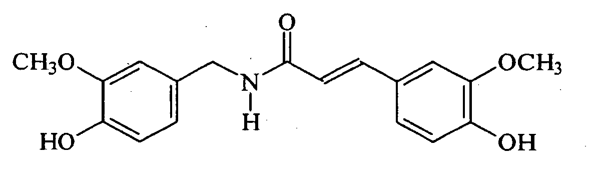 *N*-[2-(4-Hydroxy-3-methoxyphenyl)ethyl]-3-(4-hydroxy-3-methoxy-phenyl)- (*2E*)-propensäuramid (trans-Feruloylmethoxytyramin): 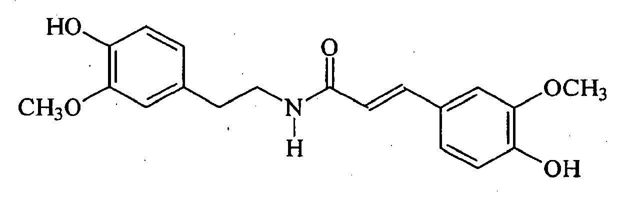 *N*-[2-(4-Hydroxy-3-methoxyphenyl)ethyl]-3-(4-hydroxy-3-methoxy-phenyl)- (*2 Z*)-propensäuramid (cis-Feruloylmethoxytyramin): 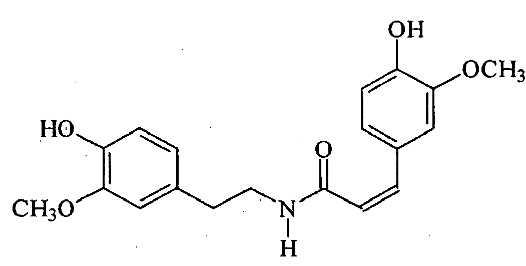 *N*-[2-(4-Hydroxy-3-methoxyphenyl)ethyl]-3-(4-hydroxy-3-methoxy-phenyl)- (-propensäuramid (Dihydroferuloylmethoxytyramin): 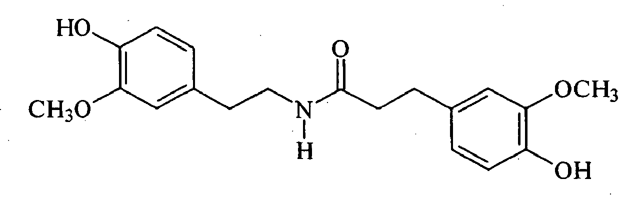 *N*-[2-(4-Diydroxyphenyl)ethyl]-3-(4-hydroxy-3-methoxy-phenyl)- (*2E*)- propensäuramid (trans-Feruloylmethoxytyramin): 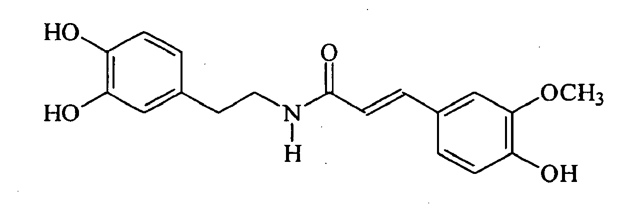 *N*-[2-(3,4-Diydroxyphenyl) ethyl]-3-(4-hydroxy-3-methoxy-phenyl)- (*2Z*)- propensäuramid (cis-Feruloyldopamin): 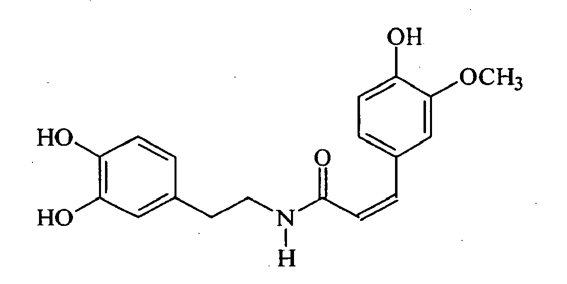 *N*-[2-(4-Hydroxyphenyl)ethyl]-3-(3,4 dihydroxyphenyl)- (*2E*)- propensäuramid (transCaffeoyltyramin): 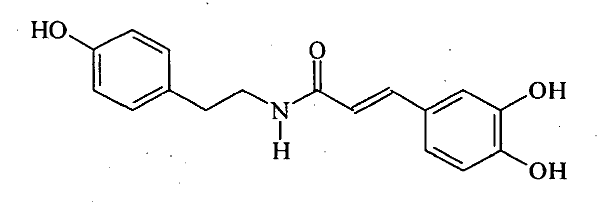 *N*-[2-(4-Hydroxyphenyl)ethyl]-3-(3,4-dihydroxyphenyl)- (*2Z*)- propensäuramid (cis Caffeoyltyramin): 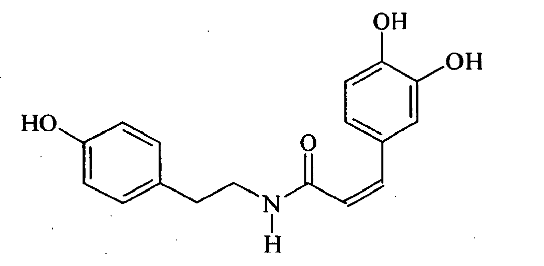 *N*-[2-(3,4-Dimethoxyphenyl) ethyl]-3-(3,4-dimethoxyphenyl)- (*2E*)-propensäuramid (cis Rubenamin): 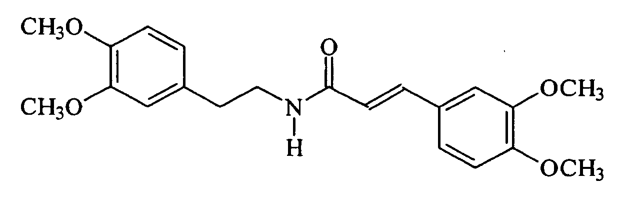 *N*-[2-(3,4-Dimethoxyphenyl) ethyl]-3-(3,4- dimethoxy-phenyl)- (*2Z*)- propensäuramid (cis Rubenamin):

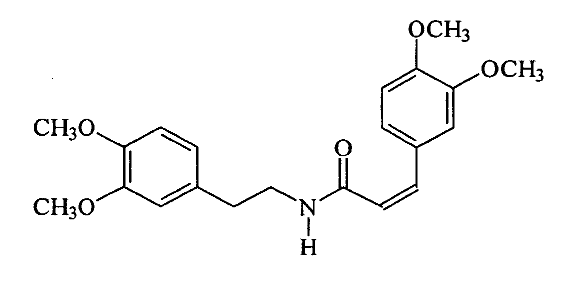

Weitere im Rahmen der vorliegenden Erfindung mit besonderem Vorzug einsetzbare Scharfstoffe sind beispielsweise Extrakte aus Naturpflanzen. Scharf schmeckende pflanzliche Extrakte können alle physiologisch unbedenklichen pflanzlichen Extrakte sein, die einen scharfen oder warmen sensorischen Eindruck hervorrufen. Bevorzugt als scharf schmeckende pflanzliche Extrakte sind beispielsweise Pfefferextrakt *(Piper ssp*., insbesondere *Piper nigrum*), Wasserpfefferextrakt (*Polygonum ssp*. ,insbesondere *Polygonum hydropiper*), Extrakte aus *Allium* ssp.(insbesondere Zwiebel und Knoblauchextrakte), Extrakte aus Rettich (*Raphanus ssp*.)*,* Meerrettichextrakte (C*ochlearia armoracia*), Extrakte aus schwarzem (*Brassica nigra*), wildem oder gelbem Senf (*Sinapis ssp*., insbesondere *Sinapis arvensis* und *Sinapis alba*), Bertramwurzel-Extrakte (*Anacyclus ssp*., insbesondere *Anacyclus pyrethrumL*.), Sonnenhutextrakte ( *Echinaceae ssp*.), Extrakten aus Szechuan-Pfeffer (*Zanthoxylum ssp*., insbesondere *Zanthoxylum piperitum*), Spilanthesextrakt (*Spilanthes ssp*., insbesondere *Spilanthes acmella*), Chiliextrakt (*Capsicum ssp*.*,* insbesondere *Capsicum frutescens* ), Paradieskörner-Extrakt ( *Aframomum* ssp.,insbesondere *Aframomum melegueta*[Rose] K. Schum.),Ingwerextrakt (*Zingiber* ssp.,insbesondere *Zingiber officinale*) und Galangaextrakt (*Kaempferia galanga* oder *Alpinia galanga*).

Eine besonders geeignete Substanz ist das aus dem Ingwerextrakt stammende Gingerol:

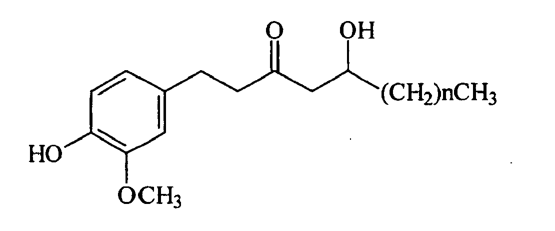

Einsetzbar ist auch N-Ethyl-p-menthan-3-carboxamid (N-Ethyl-5-Methyl-2-isopropylcyclohexancarboxamid):

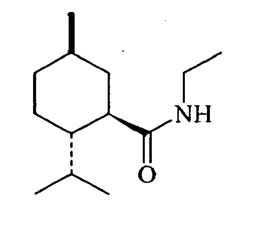

Andere scharf schmeckende oder ein Gefühl von Wärme erzeugende Substanzen können z.B. sein Capsaicin, Dihydrocapsaicin, Gingerol, Paradol, Shogaol, Piperin, Carbonsäure-N-vanillylamide, insbesondere Nonansäure-N- vanillylamid, 2-Alkensäureamide, insbesondere 2-Nonensäure-N-isobutylamid, 2- Nonensäure-N-4-hydroxy-3- methoxyphenylamid, Alkylether von 4-Hydroxy-3- methoxybenzylalkohol, insbesonders 4-Hydroxy-3-methoxybenzyl-n-butylether, Alkylether von 3- Hydroxy-4-methoxybenzylalkohol, Alkylether von 3,4- Dimethoxybenzylalkohol, Alkylether von 3-Ethoxy-4-hydroxybenzylalkohol, Alkylether von 3,4-Methylendioxybenzylalkohol, Nicotinaldehyd, Methylnicotinat, Propylnicotinat, 2-Butoxyethylnicotinat, Benzylnicotinat, 1-Acetoxychavicol, Polygodial oder Isodrimeninol.

Bevorzugte erfindungsgemäße remineralisierende Erzeugnisse sind dadurch gekennzeichnet, daß sie mindestens einen Scharfstoff aus der Gruppe der N-Alkyl-substituierte Amide von ungesättigten Carbonsäuren, vorzugsweise
a. 2E,6Z,8E-Decatriensäure-N-isobutylamid (Spilanthol) und/oder
b. 2E,4E,8Z-Decatriensäure-N-isobutylamid und/oder
c. 2E,7Z,9E-Undecatriensäure-N-isobutylamid und/oder
d. 2E,4Z-Decadiensäure-N-isobutylamid (cis-Pellitorin) und/oder
e. 2E,4E-Decadiensäure-N-isobutylamid (trans-Pellitorin) und/oder
f. Ferulasäure-N-Vanillylamid und/oder
g. *N*-[2-(4-Hydroxy-3-methoxyphenyl)ethyl]-3-(4-hydroxy-3-methoxy-phenyl)- (2*E*)-propensäureamid (trans-Feruloylmethoxytyramin) und/oder
h. *N*-[2- (4-Hydroxy-3-methoxyphenyl)ethyl]-3-(4-hydroxy-3-methoxy-phenyl)-(2 *Z*)-propensäureamid (cis-Feruloylmethoxytyramin) und/oder
i. *N*-[2-(4- Hydroxy-3-methoxyphenyl)ethyl]-3-(4-hydroxy-3-methoxy-phenyl)-propansäureamid (Dihydroferuloylmethoxytyramin) und/oder
j. *N*-[2-(3,4- Dihydroxyphenyl)ethyl]-3-(4-hydroxy-3-methoxy-phenyl)-(2*E*)-propensäureamid (trans-Feruloyldopamin) und/oder
k. *N*-[2-(3,4-Dihydroxyphenyl) ethyl]-3-(4-hydroxy-3-methoxy-phenyl)-(2*Z*)-propensäureamid (cis- Feruloyldopamin) und/oder
l. *N*-[2-(4-Hydroxyphenyl)ethyl]-3-(3,4- dihydroxyphenyl)-(2*E*)-propensäureamid (trans-Caffeoyltyramin) und/oder
m. *N*-[2-(4-Hydroxyphenyl)ethyl]-3-(3,4-dihydroxyphenyl)-(2*Z*)- propensäureamid (cis-Caffeoyltyramin) und/oder
n. *N*-[2-(3,4-Dimethoxyphenyl) ethyl]-3-(3,4-dimethoxyphenyl)-(2*E*)-propensäureamid (trans- Rubenamin) und/oder
o. *N*-[2-(3,4-dimethoxyphenyl)ethyl]-3-(3,4- dimethoxy-phenyl)-(2*Z*)-propensäureamid (cis-Rubenamin) enthalten.

Zusätzlich zu den genannten Scharfstoffen oder an ihrer Stelle können auch weitere scharf schmeckende und/oder ein Gefühl von Wärme erzeugende Substanzen in die erfindungsgemäßen Erzeugnisse eingearbeitet werden.

Als besonders geeignet haben sich im Rahmen der vorliegenden Erfindung alkylsubstituierte Dioxane der Formel 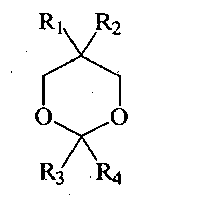 erwiesen, in der R1 und R2 unabhängig voneinander ausgewählt sind aus -H, -CH₃, -CH₂CH₃ und R3 und R4 unabhängig voneinander ausgewählt sind aus -H, -CH₃, -CH₂CH₃, -CH₂CH₂CH₃, CH(CH₃)₂

Als weiterhin besonders geeignet haben sich im Rahmen der vorliegenden Erfindung Phenylester der Formel 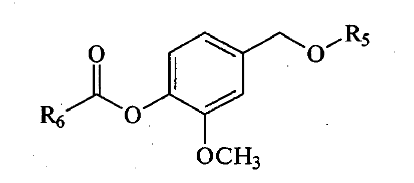 erwiesen, in der R5 für -CH₃ oder einen geradkettigen oder verzweigten Alkyl- oder Alkenylrest mit 2 bis 8 Kohlenstoffatomen und R6 für -CH₃ oder einen geradkettigen oder verzweigten Alkyl- oder Alkenylrest mit 2 bis 8 Kohlenstoffatomen oder eine Alkoxygruppe mit 1 bis 3 Kohlenstoffatomen steht Als weiterhin besonders geeignet haben sich im Rahmen der vorliegenden Erfindung Carvonacetale der Formel 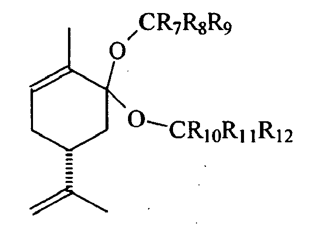 erwiesen, in der R7 bis R12 unabhängig voneinander ausgewählt sind aus -H, -CH₃, -CH₂CH₃,

-CH₂CH₂CH₃, -CH(CH₃)₂, -CH₂ CH₂CH₂CH₃, -CH₂CH(CH₃)₂, -CH(CH₃)CH₂CH₃, _C(CH₃)₃ oder R9 und R10 zusammen eine chemische Bindung oder eine Gruppe -(CR13R14)ₓ bedeuten, worin x für die Werte 1 oder 2 steht und R13 und R14 unabhängig voneinander ausgewählt sind aus -H, -CH₃, -CH₂CH₃, -CH₂CH₂CH₃, -CH(CH₃)₂, -CH₂ CH₂CH₂CH₃, -CH₂CH(CH₃)₂, -CH(CH₃)CH₂CH₃, _ C(CH₃)₃

## Patentansprüche

1. Mund- und Zahnpflege- und -reinigungsmittel, enthaltend - bezogen auf sein Gewicht -
a) 0,1 bis 1 Gew.-% Calcium-Glycerophosphat;
b) 3 bis 7 Gew.-% mindestens eines Kaliumsalzes,
wobei das Mittel weniger als 60 Gew.-% Wasser enthält.

2. Mund- und Zahnpflege- und -reinigungsmittel nach Anspruch 1, **dadurch gekennzeichnet, daß** es 0,11 bis 0,75 Gew.-% und insbesondere 0,12 bis 0,5 Gew.-% Calcium-Glycerophosphat enthält.

3. Mund und Zahnpflege- und -reinigungsmittel nach einem der Ansprüche 1 oder 2, **dadurch gekennzeichnet, daß** es Calciumsalze der Glycerophosphorsäuren der Formeln (Ia) und (Ib) enthält
HO-CH₂-CH(OH)-CH₂-OP(O)O₂²⁻ Ca²⁺ (Ia)
HO-CH₂-CH(OP(O)O₂²⁻)-CH₂-OH Ca²⁺ (Ib),
wobei das Gewichtsverhältnis der Calciumsalze der Formeln (Ia) zu (Ib) oberhalb von 50:50, vorzugsweise oberhalb von 60:40, besonders bevorzugt oberhalb von 70:30 und insbesondere oberhalb 80:20 liegt.

4. Mund und Zahnpflege- und -reinigungsmittel nach einem der Ansprüche 1 bis 3, **dadurch gekennzeichnet, daß** es 4 bis 6 Gew.-% Kaliumsalz(e) enthält.

5. Mund und Zahnpflege- und -reinigungsmittel nach einem der Ansprüche 1 bis 4, **dadurch gekennzeichnet, daß** es ausschließlich Kaliumsalz(e) enthält, welche(s) bei 20°C eine Löslichkeit in destilliertem Wasser oberhalb von 200 g/l, vorzugsweise oberhalb von 250 g/l und insbesondere oberhalb von 300 g/l aufweisen.

6. Mund und Zahnpflege- und -reinigungsmittel nach einem der Ansprüche 1 bis 5, **dadurch gekennzeichnet, daß** das Gewichtsverhältnis zwischen Calcium-Glycerophosphat und der Gesamtmenge an Kaliumsalz(en) 1 : 1000 bis 1 : 1, vorzugsweise 0,5 : 100 bis 75 : 100, weiter bevorzugt 1 : 100 bis 50 : 100,noch weiter bevorzugt 1,5 : 100 bis 10 : 100 und insbesondere 2 : 100 bis 3 : 100 beträgt.

7. Mund und Zahnpflege- und -reinigungsmittel nach einem der Ansprüche 1 bis 6, **dadurch gekennzeichnet, daß** es weniger als 55 Gew.-%, besonders bevorzugt weniger als 50 Gew.-% und insbesondere weniger als 45 Gew.-% Wasser enthält.

8. Mund und Zahnpflege- und -reinigungsmittel nach einem der Ansprüche 1 bis 7, **dadurch gekennzeichnet, daß** es zur Verbesserung der entzündungshemmenden Eigenschaften eine Kombination von
- Panthenol oder einem Salz der Pantothensäure und
- Retinol oder einem Derivat davon
enthält.

9. Mund- und Zahnpflege- und -reinigungsmittel nach einem der Ansprüche 1 bis 8, **dadurch gekennzeichnet, daß** es 0,01 bis 1 Gew.-%, vorzugsweise 0,025 bis 0,5 Gew.-% und insbesondere 0,05 bis 0,2 Gew.-% Eugenol und/oder Nelkenknospenöl enthält.

10. Mund und Zahnpflege- und -reinigungsmittel nach einem der Ansprüche 1 bis 9, **dadurch gekennzeichnet, daß** es - bezogen auf sein Gewicht - 10 bis 60 Gew.-%, vorzugsweise 12,5 bis 50 Gew.-%, besonders bevorzugt 15 bis 45 Gew.-% und insbesondere 20 bis 40 Gew.-% mindestens eines mehrwertigen Alkohols aus der Gruppe Sorbit und/oder Glycerin und/oder 1,2-Propylenglycol enthält.

11. Mund- und Zahnpflege- und -reinigungsmittel gemäß einem der Ansprüche 1 bis 10, **dadurch gekennzeichnet, daß** es zusätzlich Putzkörper, vorzugsweise Kieselsäuren, Aluminiumhydroxid, Aluminiumoxid, Calciumpyrophosphat, Kreide, Dicalciumphosphat-dihydrat (CaHPO₄ ·2H₂O), Natriumaluminiumsilikate, insbesondere Zeolith A, organische Polymere, insbesondere Polymethacrylate oder Gemische dieser Reibkörper,vorzugsweise in Mengen von 1 bis 30 Gew.%, vorzugsweise von 2,5 bis 25 Gew.% und insbesondere von 5 bis 22 Gew.%, jeweils bezogen auf das gesamte Mittel, enthält.

12. Mund- und Zahnpflege- und -reinigungsmittel nach einem der Ansprüche 1 bis 11, **dadurch gekennzeichnet, dass** es zusätzlich Phosphat(e), vorzugsweise Alkalimetallphosphat(e) und insbesondere Natriumtripolyphosphat, vorzugsweise in Mengen von 1 bis 10 Gew.-%, besonders bevorzugt von 2 bis 8 Gew.-% und insbesondere von 3 bis 7 Gew.-%, jeweils bezogen auf das gesamte Mittel, enthält.

13. Mund- und Zahnpflege- und -reinigungsmittel nach einem der Ansprüche 1 bis 12, **dadurch gekennzeichnet, daß** es zusätzlich Antiplaque-Wirkstoffe, vorzugsweise p-Hydroxybenzoesäuremethyl-, -ethyl- oder propylester, Natriumsorbat, Natriumbenzoat, Bromchlorophen, Triclosan, Hexetidine, Phenyl- Salicylsäureester, Biguanide z. B. Chlorhexidin, Thymol, vorzugsweise in Mengen von 0,05 bis 5 Gew.%, vorzugsweise von 0,10 bis 2,5 Gew.% und insbesondere von 0,30 bis 1,5 Gew.%, jeweils bezogen auf das gesamte Mittel, enthält.

14. Mund- und Zahnpflege- und -reinigungsmittel nach einem der Ansprüche 1 bis 13, **dadurch gekennzeichnet, daß** es zusätzlich Antikaries-Wirkstoffe, vorzugsweise Fluorverbindung(en), insbesondere Natriumfluorid, Kaliumfluorid, Natriummonofluorphosphat, Zinkfluorid, Zinnfluorid und Natriumfluorosilikat, vorzugsweise in Mengen von 0,01 bis 5 Gew.%, vorzugsweise von 0,1 bis 2,5 Gew.% und insbesondere von 0,2 bis 1,1 Gew.%, jeweils bezogen auf das gesamte Mittel, enthält.

15. Mund- und Zahnpflege- und -reinigungsmittel nach einem der Ansprüche 1 bis 14, **dadurch gekennzeichnet, daß** es 0,2 bis 20 Gew.-%, vorzugsweise 0,4 bis 14 Gew.-%, besonders bevorzugt 0,5 bis 3 Gew.-% und insbesondere 0,6 bis 2 Gew.-% mindestens eines bioaktiven Glases enthält.

## Claims

1. Oral and tooth care and cleaning agent which contains, based on its weight,
a) 0.1 to 1% by weight of calcium glycerophosphate;
b) 3 to 7% by weight of at least one potassium salt,
wherein the agent contains less than 60% by weight of water.

2. Oral and tooth care and cleaning agent according to Claim 1, **characterized in that** it contains 0.11 to 0.75% by weight, and in particular 0.12 to 0.5% by weight, of calcium glycerophosphate.

3. Oral and tooth care and cleaning agent according to one of Claims 1 or 2, **characterized in that** it contains calcium salts of glycerophosphoric acids of formulas (Ia) and (Ib)
HO-CH₂-CH(OH)-CH₂-OP(O)O₂²⁻ Ca²⁺ (Ia)
HO-CH₂-CH(OP(O)O₂²-)-CH₂-OH Ca²⁺ (Ib),
wherein the weight ratio of the calcium salt of formula (Ia) to the calcium salt of formula (Ib) is greater than 50:50, preferably greater than 60:40, particularly preferably greater than 70:30, and in particular greater than 80:20.

4. Oral and tooth care and cleaning agent according to one of Claims 1 to 3, **characterized in that** it contains 4 to 6% by weight of potassium salt(s).

5. Oral and tooth care and cleaning agent according to one of Claims 1 to 4, **characterized in that** it contains solely potassium salt(s) which has/have a solubility of greater than 200 g/L, preferably greater than 250 g/L, and in particular greater than 300 g/L, in distilled water at 20°C.

6. Oral and tooth care and cleaning agent according to one of Claims 1 to 5, **characterized in that** the weight ratio of calcium glycerophosphate to the total quantity of potassium salt(s) is 1:1000 to 1:1, preferably 0.5:100 to 75:100, more preferably 1:100 to 50:100, even more preferably 1.5:100 to 10:100, and in particular 2:100 to 3:100.

7. Oral and tooth care and cleaning agent according to one of Claims 1 to 6, **characterized in that** it contains less than 55% by weight, particularly preferably less than 50% by weight, and in particular less than 45% by weight, of water.

8. Oral and tooth care and cleaning agent according to one of Claims 1 to 7, **characterized in that** it contains a combination of
- panthenol or a salt of pantothenic acid and
- retinol or a derivative thereof
for enhancing the anti-inflammatory properties.

9. Oral and tooth care and cleaning agent according to one of Claims 1 to 8, **characterized in that** it contains 0.01 to 1% by weight, preferably 0.025 to 0.5% by weight, and in particular 0.05 to 0.2% by weight, of eugenol and/or clove bud oil.

10. Oral and tooth care and cleaning agent according to one of Claims 1 to 9, **characterized in that** it contains, based on its weight, 10 to 60% by weight, preferably 12.5 to 50% by weight, particularly preferably 15 to 45% by weight, and in particular 20 to 40% by weight, of at least one polyhydric alcohol from the group sorbitol and/or glycerin and/or 1,2-propylene glycol.

11. Oral and tooth care and cleaning agent according to one of Claims 1 to 10, **characterized in that** it additionally contains cleaning particles, preferably silicic acids, aluminum hydroxide, aluminum oxide, calcium pyrophosphate, chalk, dicalcium phosphate dihydrate (CaHPO₄ 2H₂O), sodium aluminum silicates, in particular zeolite A, organic polymers, in particular polymethacrylates, or mixtures of these abrasive particles, preferably in quantities of 1 to 30% by weight, preferably 2.5 to 25% by weight, and in particular 5 to 22% by weight, in each case based on the overall agent.

12. Oral and tooth care and cleaning agent according to one of Claims 1 to 11, **characterized in that** it additionally contains phosphate(s), preferably alkali metal phosphate(s), and in particular sodium tripolyphosphate, preferably in quantities of 1 to 10% by weight, particularly preferably 2 to 8% by weight, and in particular 3 to 7% by weight, in each case based on the overall agent.

13. Oral and tooth care and cleaning agent according to one of Claims 1 to 12, **characterized in that** it additionally contains antiplaque active substances, preferably p-hydroxybenzoic acid methyl, ethyl, or propyl ester, sodium sorbate, sodium benzoate, bromochlorophene, triclosan, hexetidine, salicylic acid phenyl ester, or biguanides such as chlorohexidine or thymol, preferably in quantities of 0.05 to 5% by weight, preferably 0.10 to 2.5% by weight, and in particular 0.30 to 1.5% by weight, in each case based on the overall agent.

14. Oral and tooth care and cleaning agent according to one of Claims 1 to 13, **characterized in that** it additionally contains anticavity active substances, preferably fluoride compound(s), in particular sodium fluoride, potassium fluoride, sodium monofluorophosphate, zinc fluoride, tin fluoride, and sodium fluorosilicate, preferably in quantities of 0.01 to 5% by weight, preferably 0.1 to 2.5% by weight, and in particular 0.2 to 1.1% by weight, in each case based on the overall agent.

15. Oral and tooth care and cleaning agent according to one of Claims 1 to 14, **characterized in that** it contains 0.2 to 20% by weight, preferably 0.4 to 14% by weight, particularly preferably 0.5 to 3% by weight, and in particular 0.6 to 2% by weight, of at least one bioactive glass.

## Revendications

1. Agent de nettoyage et de soins bucco-dentaires, contenant, rapportés à son poids,
a) 0,1 à 1% en poids, de glycérophosphate de calcium ;
b) de 3 à 7% en poids, d'au moins un sel de potassium,
l'agent contenant moins 60% en poids d'eau.

2. Agent de nettoyage et de soins bucco-dentaires selon la revendication 1, **caractérisé en ce qu'**il contient de 0,11 à 0,75% en poids et en particulier de 0,12 au 0,5% en poids de glycérophosphate de calcium.

3. Agent de nettoyage et de soins bucco-dentaires selon la revendication 1 ou 2, **caractérisé en ce qu'**il contient des sels de calcium des acides glycérophosphoriques de formules (Ia) et (Ib)
HO-CH₂-CH(OH)-CH₂-OP(O)O₂²-Ca₂²⁺ (Ia)
HO-CH₂-CH(OP(O)O₂²-)-CH₂-OH Ca²⁺ (Ib),
le rapport en poids des sels de calcium des formules (la) sur (Ib) étant supérieur à 50:50, de préférence supérieur 60:40, en particulier de préférence supérieur à 70:30, et notamment supérieur à 80:20.

4. Agent de nettoyage et de soins bucco-dentaires selon l'une des revendications 1 à 3, **caractérisé en ce qu'**il contient de 4 à 6% en poids de sel(s) de potassium.

5. Agent de nettoyage et de soins bucco-dentaires selon l'une des revendications 1 à 4, **caractérisé en ce qu'**il contient exclusivement un ou des sels de potassium qui présentent à 20°C une solubilité dans l'eau distillée supérieure à 200 g/l, de préférence supérieure à 250 g/l et en particulier supérieure à 300 g/l.

6. Agent de nettoyage et de soins bucco-dentaires selon l'une des revendications 1 à 5, **caractérisé en ce que** le rapport en poids entre le glycérophosphate de calcium et la quantité totale de sel(s) de potassium est de 1:1000 à 1:1, de préférence de 0,5:100 à 75:100, plus préférablement de 1:100 à 50:100, encore plus préférablement de 1,5:100 à 10:100 et en particulier de 2:100 à 3:100.

7. Agent de nettoyage et de soins bucco-dentaires selon l'une des revendications 1 à 6, **caractérisé en ce qu'**il contient moins de 55% en poids, plus préférablement moins de 50% en poids, et en particulier moins de à 45% en poids d'eau.

8. Agent de nettoyage et de soins bucco-dentaires selon l'une des revendications 1 à 7, **caractérisé en ce qu'**il contient, dans le but d'améliorer les propriétés anti-inflammatoires, une combinaison de
- panthénol ou d'un sel de l'acide pantothénique et
- de rétinol ou d'un dérivé de celui-ci.

9. Agent de nettoyage et de soins bucco-dentaires selon l'une des revendications 1 à 8, **caractérisé en ce qu'**il contient de 0,01 à 1% en poids, de préférence de 0,025 à 0,5% et en particulier de 0,05 à 0,2% en poids d'eugénol et/ou d'huile de clous de girofle.

10. Agent de nettoyage et de soins bucco-dentaires selon l'une des revendications 1 à 9, **caractérisé en ce qu'**il contient, rapportés à son poids, de 10 à 60% en poids, de préférence de 12,5 à 50% en poids, plus préférablement de 15 à 45% en poids, et en particulier de 20 à 40% en poids, d'au moins un polyol dans le groupe comprenant le sorbitol et/ou le glycérol et/ou le 1,2-propylène glycol.

11. Agent de nettoyage et de soins bucco-dentaires selon l'une des revendications 1 à 10, **caractérisé en ce qu'**il contient en outre des éléments de nettoyage, de préférence de l'acide silicique, de l'hydroxyde d'aluminium, de l'alumine, du pyrophosphate de calcium, de la craie, du phosphate dicalcique dihydraté (CaHPO₄•2H₂O), des silicates d'aluminium et de sodium, et plus particulièrement de la zéolithe A, des polymères organiques, en particulier des polyméthacrylates ou des mélanges de ces éléments abrasifs, de préférence dans des quantités de 1 à 30% en poids, de préférence de 2,5 à 25% en poids et en particulier de 5 à 22% en poids, à chaque fois rapportés à la totalité de l'agent.

12. Agent de nettoyage et de soins bucco-dentaires selon l'une des revendications 1 à 11, **caractérisé en ce qu'**il contient en outre du ou des phosphates, de préférence du ou des phosphates de métal alcalin et en particulier du tripolyphosphate de sodium, de préférence dans des quantités de 1 à 10% en poids, plus préférablement de 2 à 8% en poids, et en particulier de 3 à 7% en poids, à chaque fois rapportés à la totalité de l'agent.

13. Agent de nettoyage et de soins bucco-dentaires selon l'une des revendications 1 à 12, **caractérisé en ce qu'**il contient en outre des substances anti-plaque, de préférence de l'ester méthylique, de l'ester éthylique ou du polyester de l'acide p-hydroxybenzoïque, du sorbate de sodium, du benzoate de sodium, du bromochlorophène, du triclosan, de l'hexétidine, de l'ester phénylique de l'acide salicylique, des biguanides, par exemple la chlorhexidine, le thymol, de préférence dans des quantités de 0,05 à 5% en poids, de préférence de 0,10 à 2,5% en poids et en particulier de 0,30 à 1,5% en poids, à chaque fois rapportés à la totalité de l'agent.

14. Agent de nettoyage et de soins bucco-dentaires selon l'une des revendications 1 à 13, **caractérisé en ce qu'**il contient en outre des agents anti-carie, de préférence un ou des composés fluorés, notamment le fluorure de sodium, le fluorure de potassium, le monofluorophosphate de sodium, le fluorure de zinc, le fluorure stanneux et le fluorosilicate de sodium, de préférence dans des quantités de 0,01 à 5% en poids, de préférence de 0,1 à 2,5% en poids et notamment de 0,2 à 1,1% en poids, à chaque fois rapportés à la totalité de l'agent.

15. Agent de nettoyage et de soins bucco-dentaires selon l'une des revendications 1 à 14, **caractérisé en ce qu'**il contient de 0,2 à 20% en poids, de préférence de 0,4 à 14% en poids, de façon particulièrement préférée de 0,5 à 3% en poids et en particulier de 0,6 à 2% en poids d'au moins un verre bioactif.
